# EUROPEAN PATENT SPECIFICATION

(11) **EP 3 141 582 B1**
(45) Date of publication and mention of the grant of the patent: **13.06.2018**
(21) Application number: 15184762.1
(22) Date of filing: 10.09.2015
(51) Int. Cl.: C08L 79/02, A61K 31/13

(54) **SYNTHESIS AND USE OF POLYALKYLAMINES**
SYNTHESE UND VERWENDUNG VON POLYALKYLAMINEN
SYNTHÈSE ET UTILISATION DE POLYALKYLAMINES

(43) Date of publication of application: 15.03.2017
(73) Proprietor: Karlsruher Institut für Technologie, 76131 Karlsruhe (DE)
(72) Inventor: LEVKIN, Pavel, 76344 Eggenstein (DE); DAVIDSON, Gary, 76676 Graben-Neudorf (DE); WU, Yihang, 76131 Karlsruhe (DE); LI, Linxian, Malden, MA 02148 (US)
(74) Representative: Hoppe, Georg Johannes

(56) References cited:
- EP-A1- 2 532 649
- WO-A2-2006/081396
- US-A1- 2005 164 391
- US-A1- 2006 171 956

## Description

The present invention pertains to new cationic polyalkylamines and their synthesis and use. These cationic polyalkylamines have good properties as transfection agents. They can be used to produce polymer particles, especially polymersomes, allowing the delivery of bioactive agents into cells. The simplicity of the synthesis allows the development of a combinatorial library of cationic polyalkylamines in a kit-like manner. The compounds contained in this library can be screened for particular properties in particular for the transfection of various cell lines. The invention encompasses also the use of polymer particles containing the cationic polyalkylamines as medicament.

### Background

Of the various reagents used to transfect cells with bioactive agents such as nucleic acids, those based on polymersome mediated delivery are widely acknowledged to be the most effective. This is due mostly to their efficiency and ease of use. Polymersomes are artificially prepared spherical vesicles made of amphiphilic synthetic block copolymers. To deliver the molecules to sites of action, the polymersome can fuse with cell membrane, thus delivering the polymersome contents inside a cell.

Polymersomes are used for drug delivery due to their unique properties. A polymersome encapsulates a region of aqueous solution inside a hydrophobic membrane; dissolved hydrophilic solutes cannot readily pass through the membrane. Hydrophobic chemicals can be dissolved into the membrane, and in this way polymersome can carry both hydrophobic molecules and hydrophilic molecules. Polymersomes can be combined with bioactive agents such as drugs, nucleic acids, peptides etc., and used to deliver these agents for the regulation of a cells biochemical pathway. This opens possibilities for new treatments of diseases.

Polymer carriers are promising vectors for gene delivery due to their potential of structural diversity and flexible functionality. They could enhance the complexes of nucleic acids' stability during the circulation and give a great imaging potential for researchers to reconstruct multifunctional vectors to overcome some defects of liposomes. Over the years, a large number of cationic polymers as non-viral gene carriers have sparked the researchers' interests, including polypeptides (like poly(I-lysine)), polyethylenimine (PEI) and poly(β-amino esters) (PBAEs). They generally contain a high density of amine groups, which could be protonatable at physiological pH. When mixed with negatively charged nucleic acids, they can form stable complexes (polyplex) through electrostatic interaction and entropy change.

For delivery of negatively charged nucleic acid, cationic polymers are the most effective transfection agents. Cationic polymers represent a promising class of synthetic materials for DNA delivery. To date, there are several commercialized cationic polymers but the number of cationic polymers for safe and effective delivery of genes is still limited.

Cationic polymers need to be combined with natural phospholipids (referred to as helper lipids) to form polymersomes that can be more efficiently incorporated into cell membranes. By combining polymersomes with DNA or drugs, which alone cannot diffuse through the membrane of the target cell, they can be (indiscriminately) delivered past the lipid bilayer.

Although polymer-based reagents represent the state of the art with respect to cell transfection agents, they have the following drawbacks:
1. Many cell lines (such as primary cells) cannot be effectively transfected at the moment, even with polymer-based reagents.
2. They are relatively difficult and expensive to synthesise, often resulting in high price for end-users.

As a consequence of the second point, many laboratories use less efficient, cheaper alternatives for transfection (e.g. calcium phosphate). There is a concrete requirement for new transfection agents that are easy to synthesize and which have good transfection yields for a wide variety of cell types. As an alternative, it would be helpful to dispose an easy combinatorial synthesis of transfection agents allowing the production of a variety of different compounds.

Amino lipids, their synthesis and uses thereof are known from EP 2 532 649 A1. These amino lipids can be used as transfection agents.

### Objectives of the invention

To overcome the disadvantages of the state of the art, it is an objective of the present invention to provide novel cationic polyalkylamines and a method for their synthesis. The method should be generic, economic and easy to perform. This generic method allows the development of a library of cationic polyalkylamines. Generation of such polyalkylamines libraries (containing of hundreds of different polyalkylamine molecules) greatly enhances the identification of polyalkylamines harbouring optimal transfection reagent properties.

It is a further objective of the present invention to provide polyalkylamine particles, especially polymersomes, containing said cationic polyalkylamines. In particular, these polyalkylamine particles or polymersomes should be able to deliver bioactive agents through cell membranes. Another objective is the use of said polyalkylamine particles or polymersomes for treatment of diseases.

### Description of the invention

The invention provides novel polyalkylamines (linear, branched, or cross-linked) comprising a structure according to formula (I) or consisting of a structure according to formula (I): 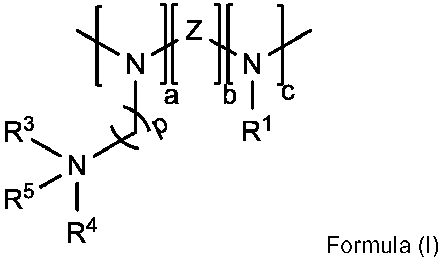 wherein
a and b are integers from 1 to 100 and c from 0 to 100;
R¹ is C₁-C₂₄ alkyl, C₂-C₂₄ alkenyl, C₂-C₂₄ alkynyl, or C₆-C₂₄ acyl, which can be optionally substituted with a C₁-C₆ hydrocarbyl group,
Z is either C1-C12 alkyls or S(=O)₂ or 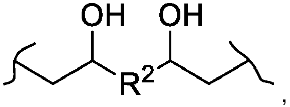 wherein the squiggly line defines the position at which this group is connected to the rest of the structure;
R² is either a mono, poly or cyclic ether or an ether which can be optionally substituted with a C1-C6 hydrocarbyl groups, or substituted with hydroxy groups,
R⁵ is either absent or is hydrogen or C₁C₁₂ alkyl to provide a quaternary amine, p is an integer from 1 to 12,
R³ and R⁴ are either the same or different and independently C₁C₁₂ alkyl, C₁C₁₂ alkenyl, or C₁C₁₂ alkynyl, wherein alkyl, alkenyl or alkynyl may be optionally substituted with a C₁-C₆ hydrocarbyl group, or R³ and R⁴ may join to form, together with the nitrogen atom to which they are bound, an optionally substituted heterocyclic ring of 3 to 10 atoms comprising 1 to 7 heteroatoms (including the nitrogen atom to which R³ and R⁴ are bound) chosen from nitrogen, thiol and oxygen, R³ and R⁴ can also connect two or more polymer chains comprising a structure of Formula (I) leading to a cross-linked or branched polymer.

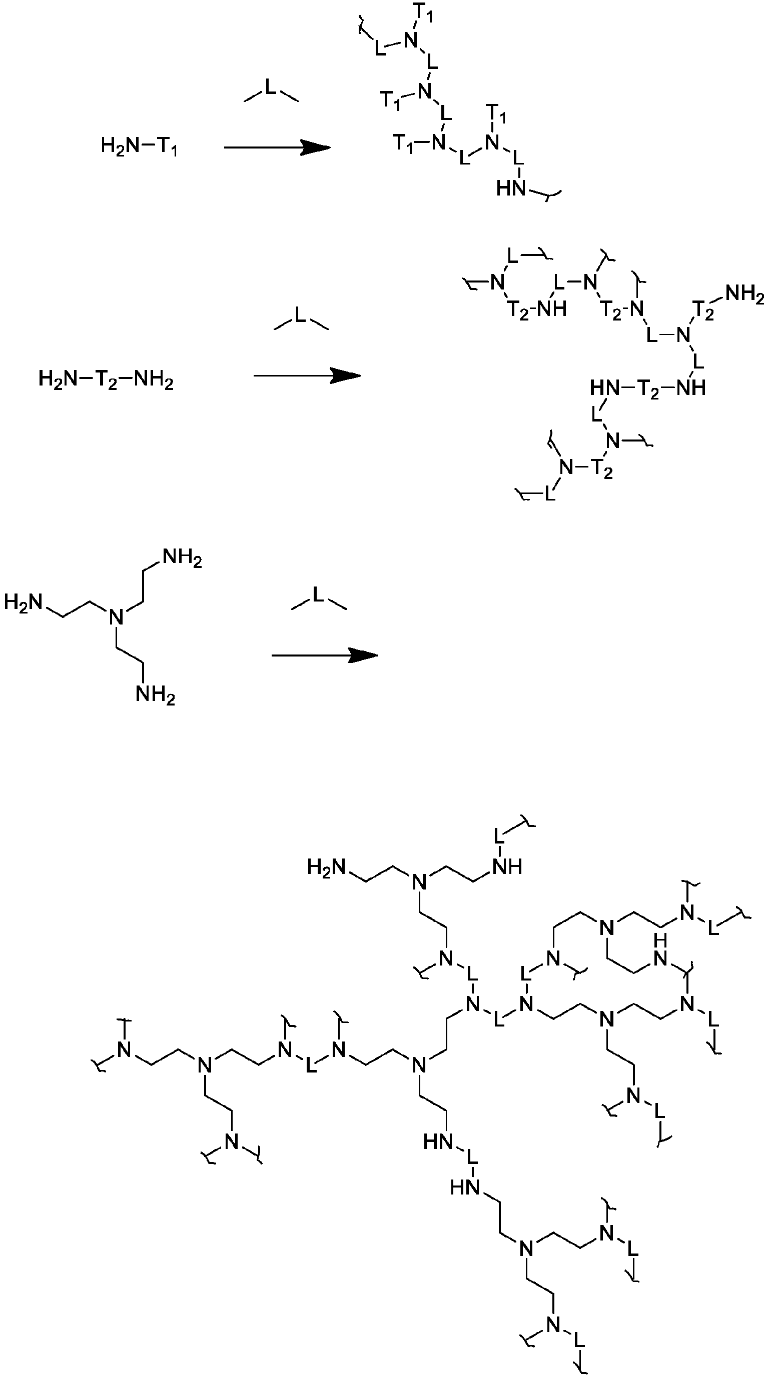

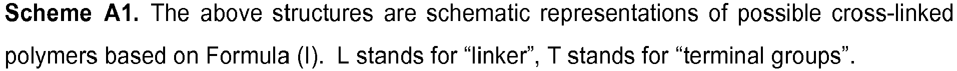

In a preferred embodiment of the invention, R¹ is C₆-C₂₄ alkyl, more preferred, R¹ and is C₆-C₁₈ alkyl.

In one embodiment c is 0, resulting in general formula (II): 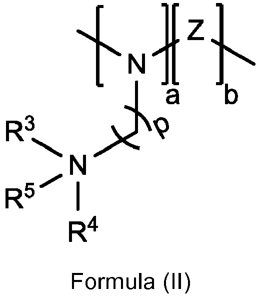 wherein Z, R³, R⁴, R⁵, p, a and b are defined as for Formula (I).

Also, the present invention provides polyalkylamines of the general formula (III): 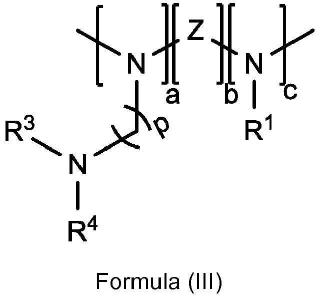 wherein
R¹ is C₆-C₁₈ alkyl
R³,R⁴, p, a, b and c are defined as for Formula (I).

In a preferred embodiment of the compounds according to formulas (I), (II) and (III), p is an integer chosen from 1, 2 or 3.

The most preferred embodiments correspond to the structures of formula (III), 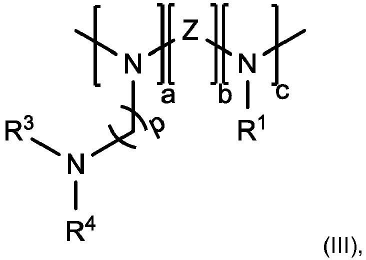 wherein
a and b are integers from 1 to 100 and c is an integer from 0 to 100;
R¹ is C₁₁-C₁₂ alkyl,
R³ and R⁴ are the same C₁-C₂ alkyls, R³ and R⁴ can also connect two or more polymer chains (III) leading to a cross-linked or branched polymers similar to those depicted in Scheme A1, p is an integer from 1 to 2.

The present invention provides a method to synthesize polyalkylamines with a structure according to Formulae (I) to (III). The method represents the parallel synthesis of large libraries of ionisable cationic polyalkylamines based on nucleophilic substitution, Michael addition, or nucleophilic addition chemistry in liquid-phase combinatorial synthesis without chromatography purification:
Reaction of dihalides, dialkenes or diepoxides of the general formula (IVa), (IVb), or (IVc) 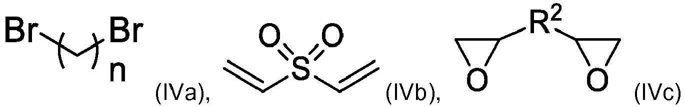 with an amine of the general formula (R³R⁴R⁵N)(CH₂)ₚX, and another amine of the general formula, NH₂-R¹,
under polymerization, to yield a compound of the general formula (Va), (Vb) or (Vc) 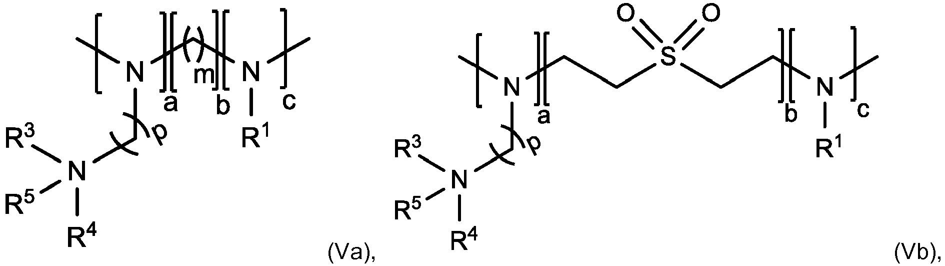 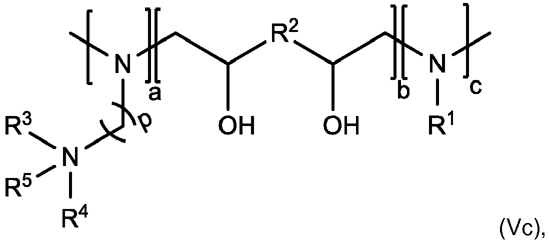 wherein
   a and b are integers from 1 to 100 and c is an integer from 0 to 100;
   n is an integer from 1 to 12;
   R² is either mono, poly or cyclic ethers or ethers which can be optionally substituted with a C1-C6 hydrocarbyl groups, or substituted with hydroxy groups;
   X is NH₂ or NH;
   p is an integer from 1 to 12;
   m is an integer from 1 to 12;
   R¹ is C₁-C₂₄ alkyl, C₂-C₂₄ alkenyl, C₂-C₂₄ alkynyl, or C₆-C₂₄ acyl, which can be optionally substituted with a C₁-C₆ hydrocarbyl group;
   R³ and R⁴ are either the same or different and independently C₁C₁₂ alkyl, C₁C₁₂ alkenyl, or C₁C₁₂ alkynyl, wherein alkyl, alkenyl or alkynyl are optionally substituted with a C₁-C₆ hydrocarbyl group, or R³ and R⁴ may join to form, together with the nitrogen atom to which they are bound, an optionally substituted heterocyclic ring of 3 to 10 atoms comprising 1 to 7 heteroatoms (including with the nitrogen atom to which R³ and R⁴ are bound) chosen from nitrogen, thiol and oxygen;
   R⁵ is either absent or is hydrogen or C₁-C₁₂ alkyl to provide a quaternary amine.
   R³ and R⁴ can also connect two or more polymer chains comprising a structure of formula (III) leading to a cross-linked or branched polymers, as described for formula (I).

This reaction is conducted via a nucleophilic substitution, Michael addition, or nucleophilic addition chemistry.

This reaction scheme is very versatile; it can be used to synthesize large libraries of cationic polyalkylamines for rapid cell-based screening assay in a very inexpensive manner. The resulting compounds all have both a hydrophobic character due to their long non-polar residues and a hydrophilic character due to their amino group. This amphiphilic character can be used to form polyalkylamine particles or polymersomes. Moreover, the amino group of these compounds confers a cationic charge which is useful for transfection agents. This library of different compounds with new characteristics can be tested easily for their transfection capacity of a wide variety of cell types.

Another embodiment of the present invention is directed to polyalkylamine particles containing a polyalkylamine comprising a structure according to formula (I), (II) or (III). Within the scope of the invention, the term 'polyalkylamine particle' means nanosized objects made of polyalkylamines which are placed into an aqueous solution. These particles are polymersomes.

In a preferred embodiment of the present invention, said nanoparticles are polymersomes containing a polyalkylamine comprising a structure according to formula (I) to (III). Within the scope of the invention polymersomes are microvesicles composed of amphipathic (amphiphilic) molecules enclosing an aqueous compartment.

Polymersome formation is not a spontaneous process. Polymersomes are formed first when polyalkylamines are placed in water and consequently form an aqueous solution in their core.

Within the scope of the invention, the term 'cells' means a generic term and encompass the cultivation of individual cells, tissues, organs, insect cells, avian cells, fish cells, amphibian cells, mammalian cells, primary cells, continuous cell lines, stem cells and/or genetically engineered cells, such as recombinant cells expressing a heterologous polypeptide or protein. Recombinant cells include, for example, cells expressing heterologous polypeptides or proteins, such as a growth factor or a blood factor.

In a preferred embodiment said polyalkylamine particles or polymersomes further contain a helper lipid. In a preferred embodiment said helper lipid is a non-cationic lipid. In a more preferred embodiment said helper-lipid is a non-cationic phospholipid. Polymersomes consisting of a mixture of cationic polyalkylamines and non-cationic (neutral) phospholipids are the most effective for nucleic acid delivery into cells. In an even more preferred embodiment said non-cationic lipid is DOPE. In a most preferred embodiment, helper lipid and polyalkylamines comprising a structure according to formula (I) to (III) are added in a 1:1 ratio to form polymersomes.

In a further preferred embodiment the polyalkylamine particles or polymersomes further comprise a sterol. Sterol, like cholesterol, is a natural component in cell membranes. It can be used to stabilise the particle, and help the integration with cell membrane.

In another embodiment of the invention, the polyalkylamine particles or polymersomes further contain a bioactive agent. Within the scope of this invention a bioactive agent is one which has a biological effect when introduced into a cell or host, for example by stimulating an immune response or an inflammatory response, by exerting enzymatic activity or by complementing a mutation, etc. bioactive agents include inter alia nucleic acids, peptides, proteins, antibodies and small molecules.
When a polymersome is used to encapsulate a drug substance either in the interior aqueous space of the polymersome the term 'polymersome drug' can be employed.

In a most preferred embodiment, the bioactive agent is a nucleic acid. In another preferred embodiment said bioactive agent is a member optionally selected from the group consisting of: an antineoplastic agent, an antibiotic, an immunomodulator, an anti-inflammatory agent, an agent acting on the central nervous system, a polypeptide or a polypeptoid.

In yet another embodiment the polyalkylamine particles or polymersomes further contains at least one polyethylene glycol (PEG)-lipid. PEG lipids help to protect the particles and their cargo from degradation in-vivo. Moreover, PEG form a protective layer over the polymersome surface and increase the circulating time in vivo. It can be used in polymersome drug delivery (PEG- polymersome).

Polyalkylamine particles or polymersomes containing a bioactive agent can be used to deliver any of a variety of therapeutic agents into cells. The present invention encompasses the in vitro use of polyalkylamine particles, especially polymersomes, as described above for delivering a bioactive agent into a cell.

Preferably said bioactive agent is a nucleic acid, including but not limited to, RNA, antisense oligonucleotide, a DNA, a plasmid, a ribosomal RNA (rRNA), a micro RNA (miRNA), transfer RNA (tRNA), a small inhibitory RNA (siRNA) or small nuclear RNA (snRNA). The bioactive agent may also be an antineoplastic agent, an antibiotic, an immunomodulator, an anti-inflammatory agent, an agent acting on the central nervous system, antigens or fragments thereof, proteins, peptides, polypeptoid, vaccines and small-molecules or mixtures thereof.

As has been set out above, polyalkylamine particles or polymersomes containing polyalkylamines as defined in the present invention are suitable to deliver bioactive agents into cells. The wide variety of different polyalkylamines which can be synthesized by the mentioned versatile synthesis method can be screened for particular characteristics that are conferred to the polymersomes. Important characteristics are for example transfection efficiency, cytotoxicity, adhesion of the agent to be delivered into the cell, stability of the polymersomes, size of the polymersomes, etc. The present method allows the creation of specifically adapted polymersomes for particular applications.

For example polyalkylamine particles (polymersomes) can be used for transfecting multicellular tissues or organisms. This offers the possibility of a novel therapeutic treatment of patients.

According to the present invention, a patient can be any mammal, preferably selected from the group consisting of human, mouse, rat, pig, cat, dog, horse, goat, cattle, and monkey and/or others. Most preferably, the patient is a human being.

An important embodiment of the present invention is the use of said polyalkylamine particles or polymersomes containing polyalkylamines according to one of formulas (I-III) for use as a medicament.

In particular, said polyalkylamine particles or polymersomes can be administered to patients for use in gene therapy, in gene vaccination, in antisense therapy or in therapy by interfering RNA. A polyalkylamine particle of the invention may also be used for the manufacture of a medicine for use in nucleic acid transfer, for example in treatment of the human or animal body by therapy, especially in the treatment of a condition caused by or related to a genetic defect or modification.

Targets for gene therapy are well known and include monogenic disorders, for example, cystic fibrosis, various cancers, and infections, for example, viral infections, for example, with HIV. For example, transfection with the p53 gene offers great potential for cancer treatment. Targets for gene vaccination are also well known, and include vaccination against pathogens for which vaccines derived from natural sources are too dangerous for human use and recombinant vaccines are not always effective, for example, hepatitis B virus, human immunodeficiency virus (HIV), hepatitis C virus (HCV) and herpes simplex virus.

Targets for anti-sense therapy are also known. Further targets for gene therapy and anti-sense therapy are being proposed as knowledge of the genetic basis of disease increases, as are further targets for gene vaccination.

A polyalkylamine particle of the invention may be used in vaccination. Thus, a polyalkylamine particle or polymersome of the invention may be used to deliver an antigen or a nucleic acid encoding an antigen. These techniques are familiar to a person skilled in the art. Examples for polymersome vaccines are described in biodegradable polymer microspheres as vaccine adjuvants and delivery systems. (Dev Biol Stand. 1998; 92:63-78) and vaccine delivery system for immunization, using biodegradable polymer microspheres (US 5569468 A).

A polyalkylamine particle of the invention may be used to elicit an immune response against a wide variety of antigens for the treatment and/or prevention of a number of conditions including, but not limited to, cancer, allergies, toxicity and infection by pathogens such as viruses, bacteria, fungi, and other pathogenic organisms.

In a preferred embodiment of the invention said polyalkylamine particle or polymersome can be used as a medicament in the treatment of a viral infection, a liver disease or disorder, or cancer. In case of liver diseases, polymersomes can be captured by the cells of the reticulo-endothelial system, which are primarily situated in the liver. The polymersome will be accumulated there.

In another aspect, the invention refers to a kit comprising at least 5, in particular at least 10, at least 100, at least 1000, or at least at least 10.000 different polyalkylamines of Formula (I), (II) or (III).

In yet another aspect, the invention refers to the use of a kit as described for screening a plurality of different polyalkylamine of Formula (I), (II) or (III) for a desired property. Such a property can be, for example, the transfection efficiency for a given cell type.

In another aspect, the invention refers to a method for screening a plurality of different polyalkylamine of Formula (I), (II) or (III) for a desired property. The method comprises bringing the plurality of different polyalkylamines into contact with a given cell type and determining which of the polyalkylamine(s) of the plurality of polyalkylamines has the desired property or shows the desired property best.

### Figures

The following figures are presented to provide a better understanding of the description of procedures and conceptual aspects of the invention.

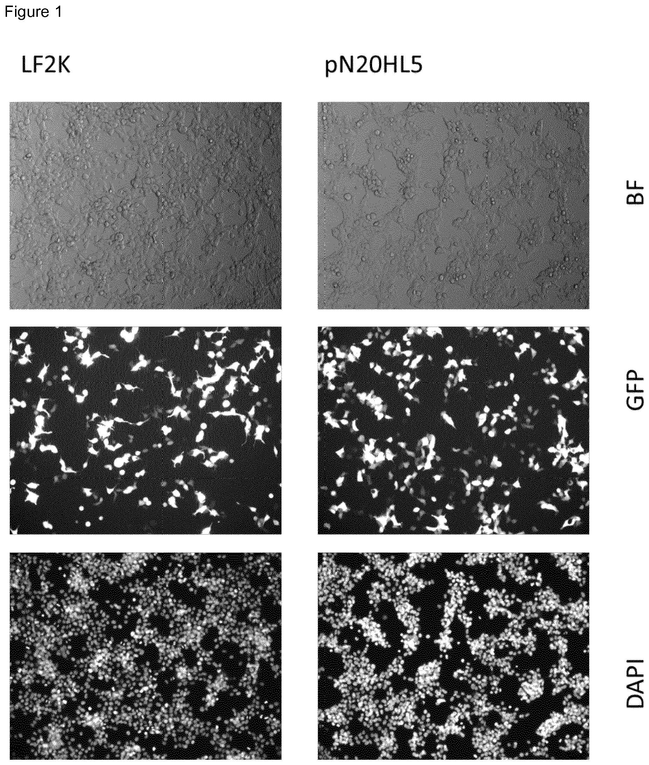

**Fig. 1****:** Comparative microscope pictures showing results of transfection by use of a polyalkylamine reagent and one commercially available transfection reagent. Images show bright field (highest panel) nuclei stained with the Hoechst (lowest panel, showing total cell number) and GFP positive cells (medium panel, transfected cells). LF2K is used as positive control.

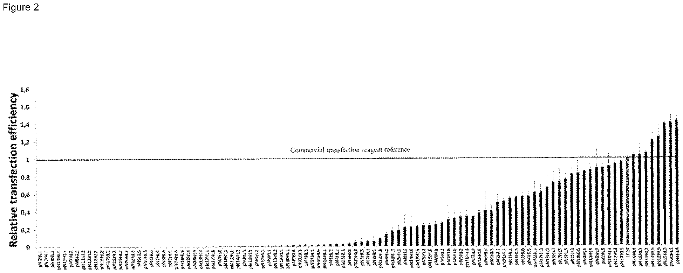

**Fig. 2****:** Graphical overview of the transfection efficiency of a library of 103 transfection reagents compared to a commercially available reagent (Lipofectamine 2000). *In vitro* HEK 293T cells transfection screening of 103 polyalkylamine reagents with eGFP-pDNA. Transfection efficiency was determined by calculating the ratio of fluorescent to total cells after transfection with a plasmid containing the green fluorescent protein (GFP) gene. Relative transfection efficiency is a ratio of the transfection efficiency of a polymer to that of the positive control (LF2K).

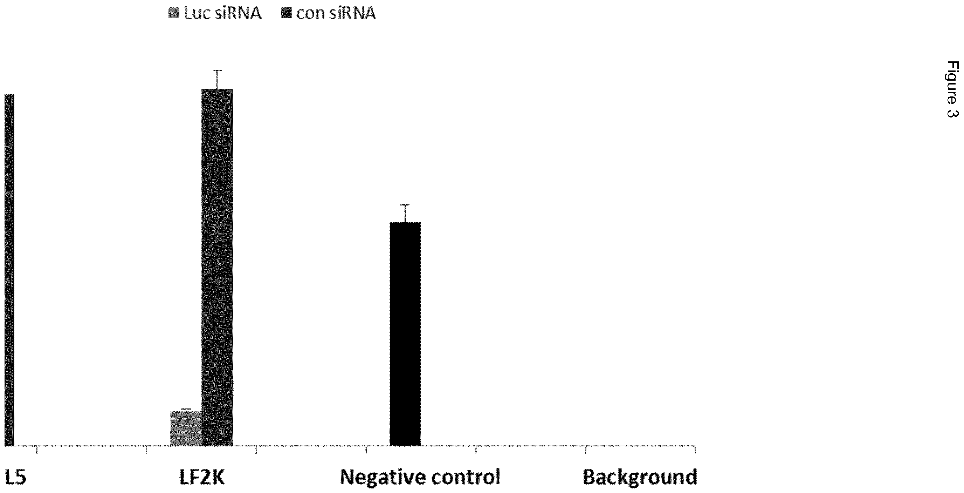

**Fig. 3****:** siRNA gene silencing in U87Luc cells.
   Expression of luciferase protein from total lysates of U87Luc cells transfected with the indicated siRNA molecules and cultured for 48 hours using either LF2K as reference or one polyalkylamine reagent pN19HL5. Untreated cells (NC) were used as the negative control.

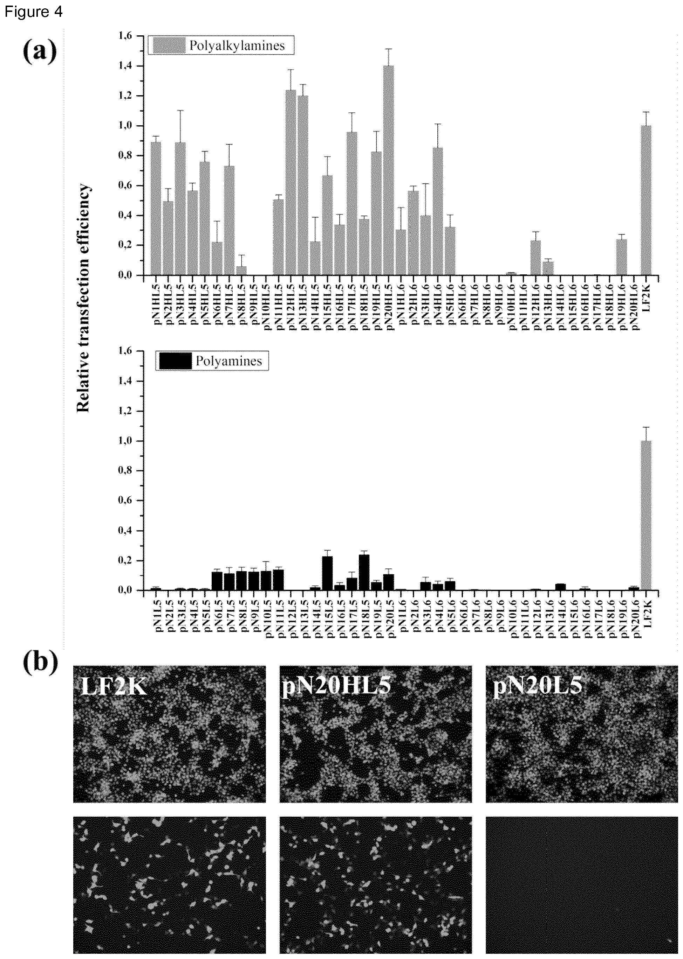

**Fig. 4****:** *In vitro* HEK 293T cells transfection screening of 40 polyamines 40 polyalkylamines with eGFP-pDNA. Relative transfection efficiency of 40 polyamines 40 polyalkylamines (a) at one DNA/polymer monomer ratios was shown. Transfection efficiency was determined by calculating the ratio of fluorescent to total cells after transfection with a plasmid containing the green fluorescent protein (GFP) gene. Relative transfection efficiency is a ratio of the transfection efficiency of a polymer to that of the positive control (LF2K). (b) Examples of fluorescent microscopy images of cells transfected with two typical polymers (pN20HL5 and pN20L5) from the library. Images show nuclei stained with the Hoechst (upper panel, showing total cell number) and GFP positive cells (lower panel, transfected cells). LF2K is used as positive control.

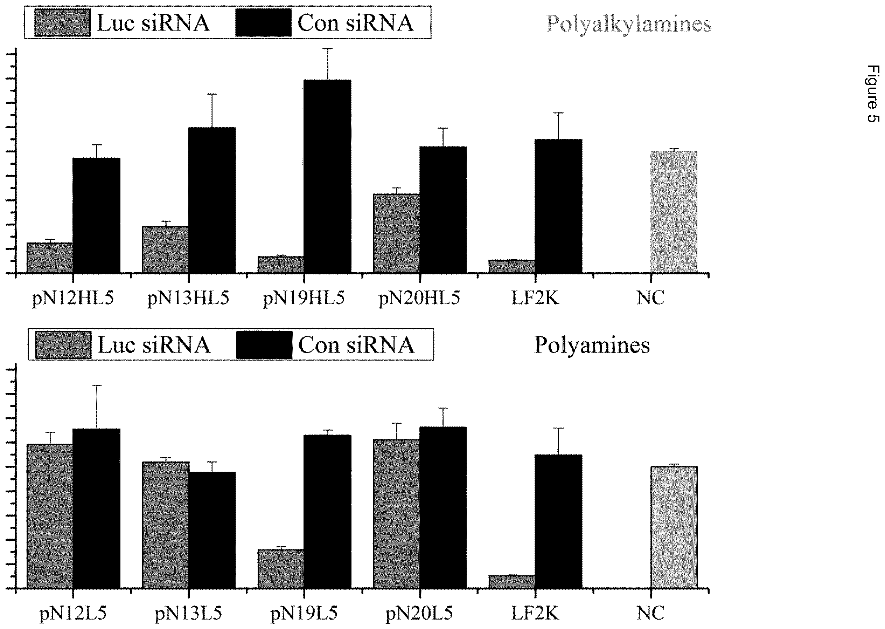

**Fig. 5****:** Expression of luciferase protein from total lysates of U87Luc cells transfected with the indicated siRNA molecules and cultured for 48 hours using either LF2K as reference or polymer particles. Untreated cells (NC) were used as the negative control.

### Examples

The following examples are presented to provide a better understanding of the description of procedures and conceptual aspects of the invention.

### Synthesis

### Example 1: Synthesis and characterization of 1^{st} polymer library

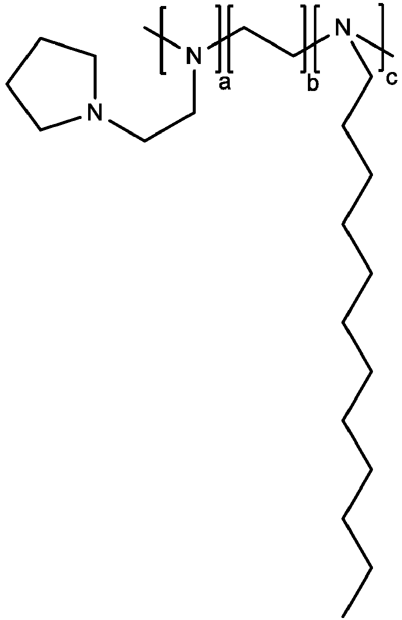

a and b are integers from 1 to 100 and c is an integer from 0 to 100;
The polyalkylamine was synthesized as follows: Cs₂CO₃ (3 mmol) in ethanol (3 mL) was placed in a 20-mL vial. An amine (0.5 mmol), Dodecylamine (0.5 mmol), and either 1,2-Dibromoethane, or 1,6-Dibromohexane, or 1,12-Dibromododecane (1 mmol) were added to the vial. The resulting mixture was shaken (180 rpm) at 40 °C for 20 h. After cooling, the Cs₂CO₃ was removed by addition of 1 mL of H₂O and CH₂Cl₂ to the product. The supernatant was removed. The residuum was evaporated to give final product that was further used.

Synthesis of the compounds of the examples 2 to 5 was carried out similarly to example 1. The resulting compounds are summarized in Table 1:

**Table 1: Examples of synthesized compounds 1-5.**

| **Example** | **Compound** | **Compound name** |
|---|---|---|
| **1** | 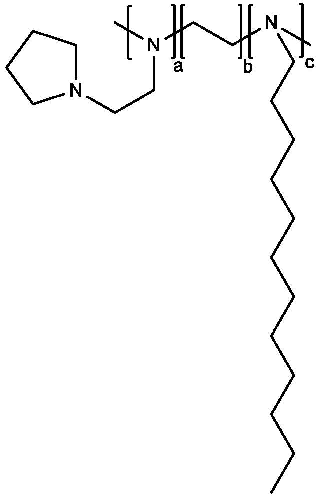 | pN1HL1 |
| **2** | 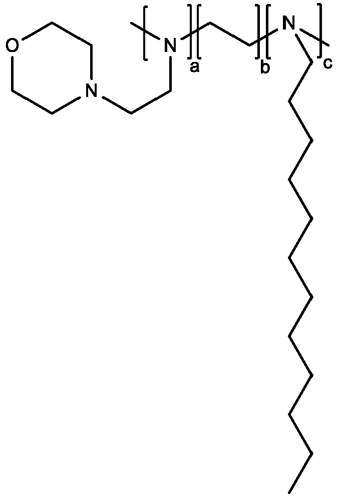 | pN2HL1 |
| **3** | 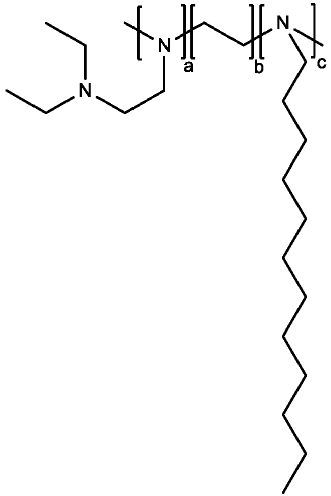 | pN3HL1 |
| **4** | 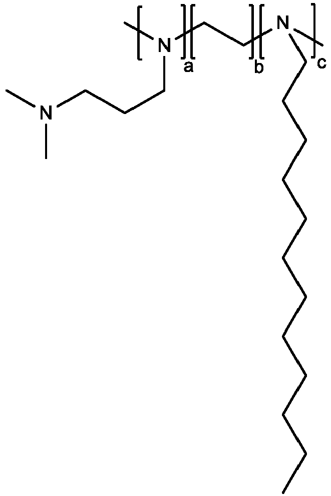 | pN4HL1 |
| **5** | 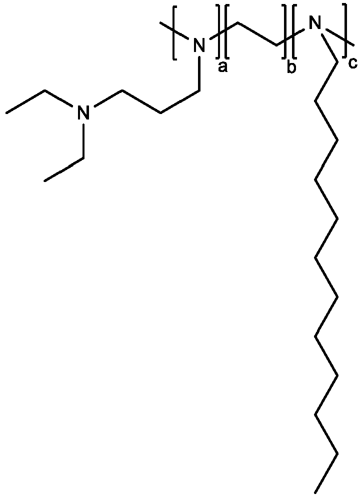 | pN5HL1 |

### Example 6: Synthesis and characterization of 2^{nd} polymer library

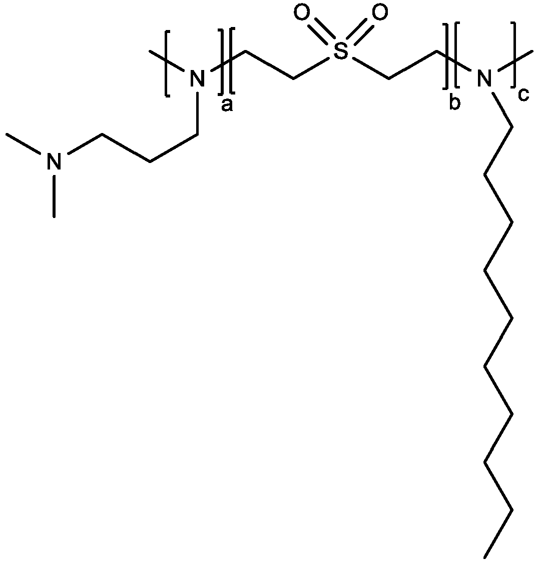

a and b are integers from 1 to 100 and c is an integer from 0 to 100;
The polyalkylamine was synthesized as follows, an amine (0.5 mmol), Divinyl sulfone (1 mmol), and either Decylamine, or Undecylamine, or Dodecylamine (0.5 mmol) were combined in a 20 ml glass vial of 2 ml DMSO and vortexed. The solutions were then shaked (700 rpm) at 50 °C for 5 days.

Synthesises according to the examples 7 to 8 were carried out similarly to example 6. The resulting compounds are resumed in table 2:

**Table 2: Examples of synthesized compounds 6-8.**

| **Example** | **Compound** | **Compound number** |
|---|---|---|
| **6** | 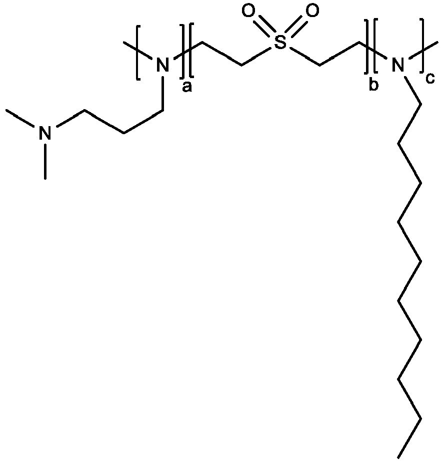 | pN4HL4 |
| **7** | 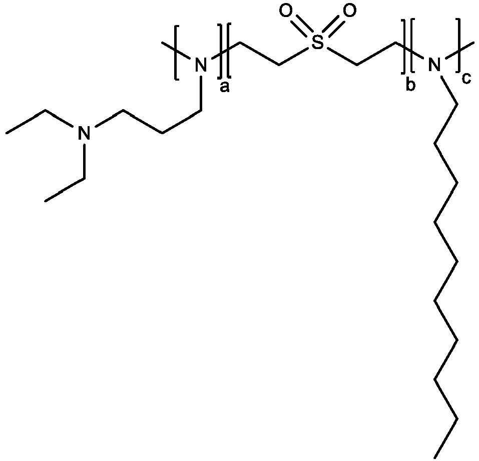 | pN5HL4 |
| **8** | 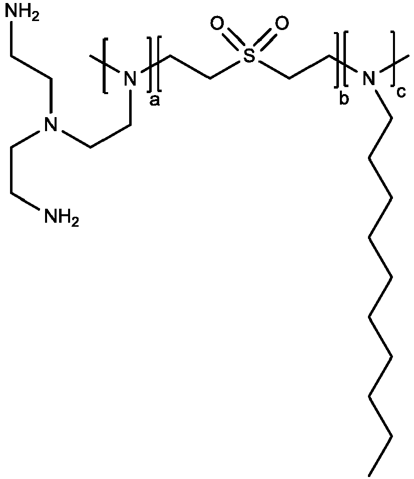 | pN15HL4 |

### Example 9: Synthesis and characterization of 3^{rd} polymer library - poly(beta-amino alcohol)s (PBAAs-1)

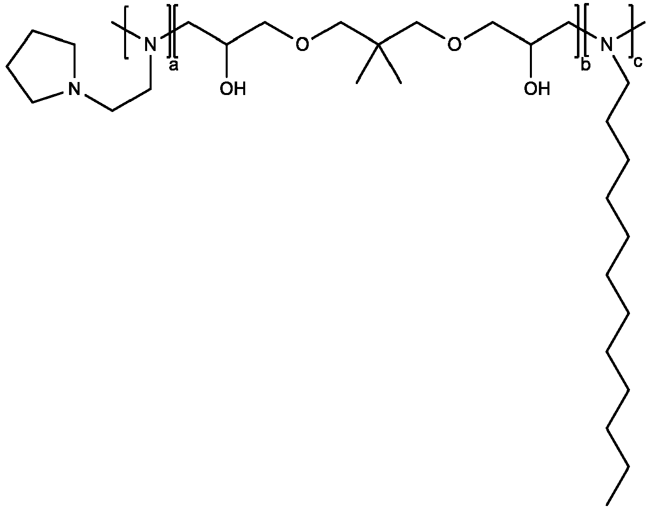

a and b are integers from 1 to 100 and c is an integer from 0 to 100;
The polyalkylamine was synthesized as follows, an amine (0.5 mmol), a diglycidyl ether, either Neopentyl glycol diglycidyl ether or 1,4-Cyclohexanedimethanol diglycidyl ether (1 mmol) and Dodecylamine (0.5 mmol) were combined in a 20 ml glass vial of 2 ml ethanol and vortexed. The solutions were then shaked (180 rpm) for 24 h at room temperature.

Synthesis of the compounds of the examples 10-68 were carried out similarly to example 9. The resulting compounds are summarized in Table 3:

**Table 3: Examples of synthesized compounds 9-13.**

| **Example** | **Compound** | **Compound number** |
|---|---|---|
| **9** | PBAAs-1 | pN1HL5 |
| | 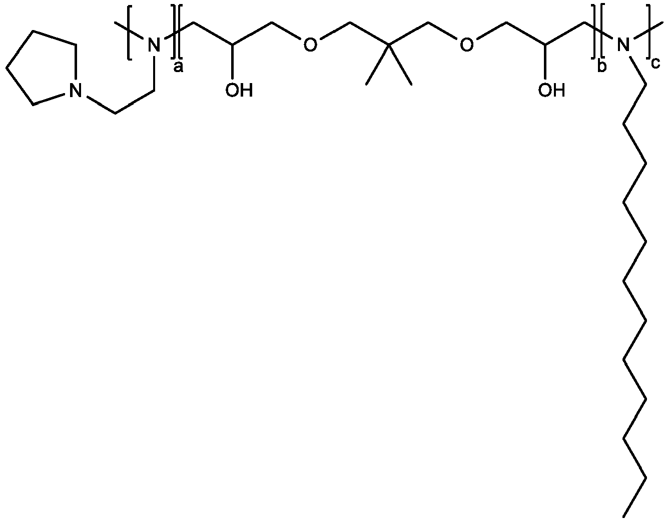 | |
| **10** | PBAAs-2 | pN2HL5 |
| | 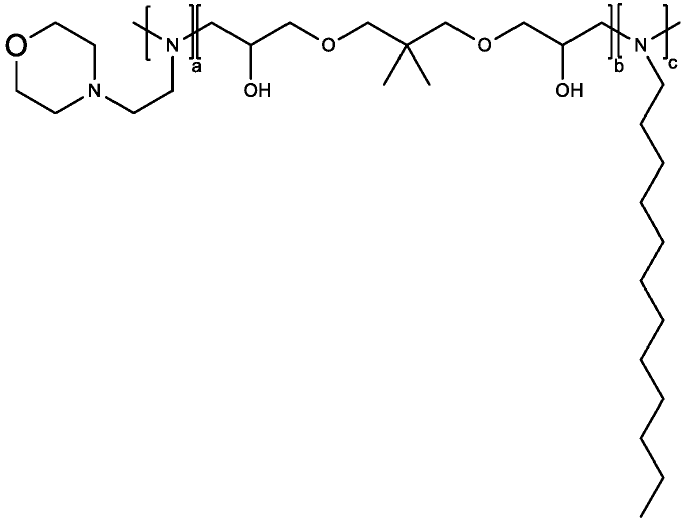 | |
| **11** | PBAAs-3 | pN3HL5 |
| | 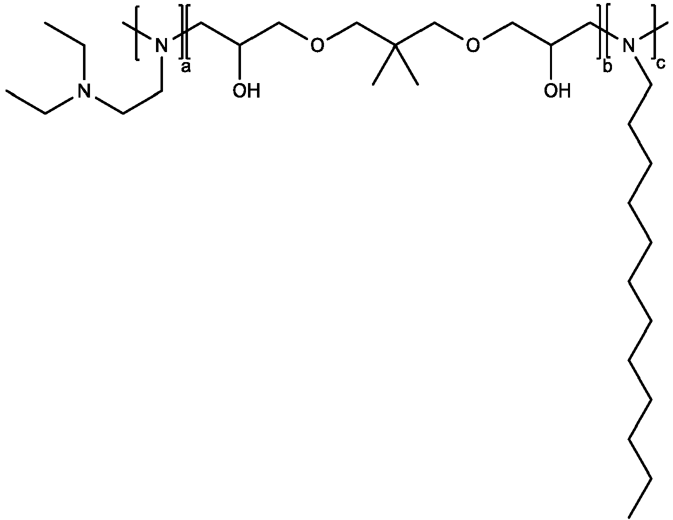 | |
| **12** | PBAAs-4 | pN4HL5 |
| | 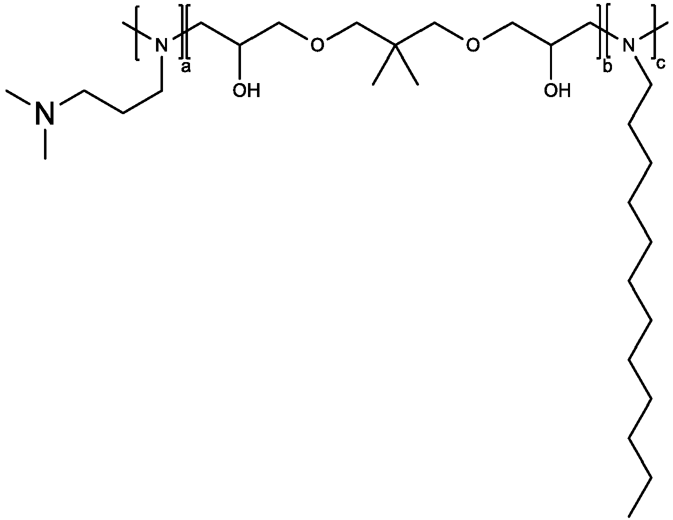 | |
| **13** | PBAAs-5 | pN5HL5 |
| | 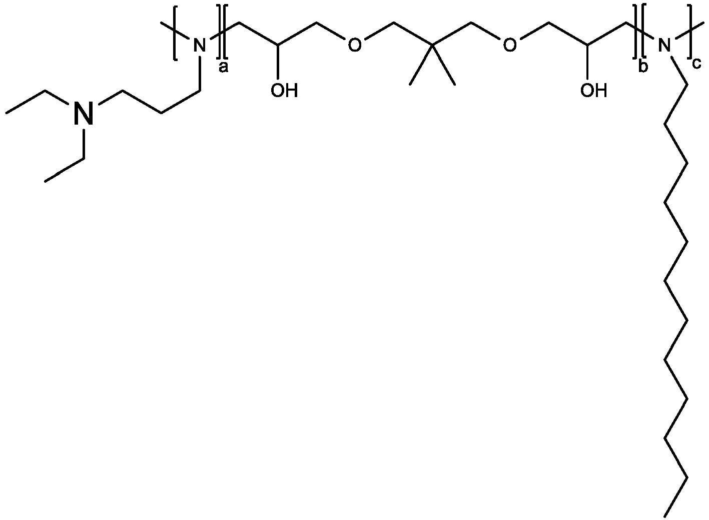 | |

### Screening of the cationic polyalkylamines for cell transfection

### Example 14: Initial determination of optimal polyalkylamine ratios for cell transfection.

The well documented HEK 293T cell line is used for examples 14 and 15.

The natural phospholipid dioleolylphosphatidylethanolamine (DOPE - structure shown below) was selected as the required co-lipid (also termed helper lipid). It is required not for the stability of polymersomes per se, but rather the breakdown of the lipid membranes in the endocytic compartment (endosomes) of cells, allowing release of the bioactive agent to the cytosol and/or nucleus. Basically, it is required for the desired effect for stable polymersome formation in combination with our cationic polyalkylamine. DOPE was mixed with a representative cationic polyalkylamine (structure shown below) in different ratios. Polyalkylamine and DOPE were dissolved in ethanol at 50 mg/ml and combined to a final volume of 30 µl.

### DOPE (co-lipid):

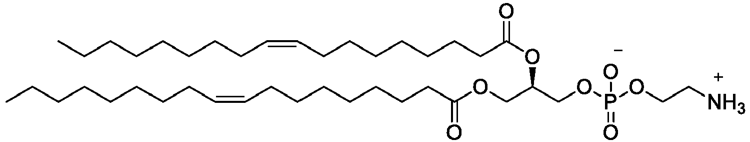

### PBAAs-1 as representative novel cationic polyalkylamine:

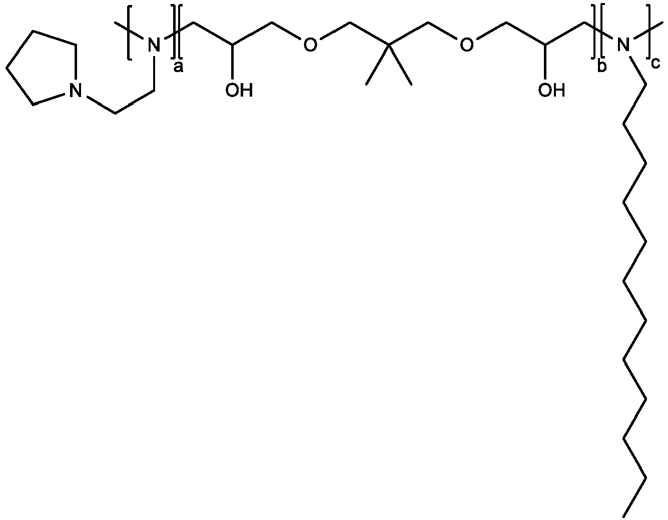

a and b are integers from 1 to 100 and c is an integer from 0 to 100;

**Table 4: PBAAs-1: DOPE ratios tested**

| **PBAAs-1** | **DOPE** |
|---|---|
| 0 | 1 |
| 1 | 3 |
| 1 | 2 |
| 1 | 1 |
| 2 | 1 |
| 3 | 1 |
| 1 | 0 |

These 30 µl ethanol mixtures were then added to 70 µl of 0.2 mol/l Sodium acetate buffer (pH 5.0) with constant vortexing for 30 s, followed by sonication for 5 min to form polymersomes. Final polymer content is 2 mg/ml. This final 2 mg/ml polymersome sample is referred to as the "polyalkylamine reagent".

0.1 µl, 0.2 µl, 0.3 µl, 0.4 µl and 0.5 µl of the polyalkylamine reagents of Table 4 were combined with either 50 ng or 100 ng plasmid DNA (comprising the pCS-LacZ and EGFP plasmids at a ratio of 5:1, respectively) and mixed with cells as described below:
(amounts shown are for one well of a 96-well culture plate)
**1.** 0.1 µl-0.5 µl polyalkylamine reagent diluted in 10 µl of 50 mmol/l sodium acetate buffer, pH 5.0
**2.** After 2-5 min incubation, added diluted polyalkylamine reagent from (1) to either 50ng or 100ng plasmid DNA (DNA dissolved in 10 µl of 50 mmol/l sodium acetate buffer, pH 5.0).
**3.** Samples were left at RT for 30 min to form polyalkylamine/DNA transfection complexes. As DNA is negatively charged, it associates non-specifically with the positively charged head groups of the cationic polyalkylamine in the polymersomes.
**4.** After 30 min, 50 µl of freshly suspended HEK 293 cells (approximately 50,000 cells, in DMEM culture medium supplemented with 10 % foetal calf serum) were added to the polyalkylamine/DNA complexes, mixed with pipette action and 65 µl of the cells + polyalkylamine/DNA complexes added to a single 96-well.

To assess the ability of the polyalkylamine mixtures to deliver the plasmid DNA into cells, microscopy was used to visualize fluorescence emitted by the green fluorescent protein (GFP) 20-24 hours after initial transfection. The GFP protein is encoded by the GFP plasmid.

### RESULTS:

A optimal ratio of polyalkylamine: DOPE was identified as 2 : 1 and the optimal polyalkylamine reagent: DNA ratio was 0.4 µl polyalkylamine reagent per 75 ng DNA. These conditions were therefore used for the primary screen to identify the polyalkylamine reagents with highest cell transfection efficiency and lowest cell toxicity, as described in example 44 below.

### Example 15: Primary screen using novel polyalkylamine reagents

Cell line: HEK 293 cells
Screen format: 96-well
Detection (read-out): GFP fluorescence relative to total cell number (total cell number assessed using the nuclear dye, Hoechst - see **Fig. 1**)

A commercially available liposomal transfection reagent was used as a reference (reference reagent) according to the manufacturers' instructions, see **Fig.1****.**

### Method:

All steps performed in 96 tube/plate format using 8- or 12-channel multipipettes. Amounts shown are for duplicate (2x) wells of a 96-well plate.
1. 0.8 µl polyalkylamine reagents diluted in 20 µl 50 mmol/l NaOAc buffer, pH 5.0.
2. Diluted polyalkylamine reagents from (1) were added to 150ng DNA (25ng EGFP + 125 ng pCS-LacZ plasmids) in 20 µl NaOAc buffer, pH 5.0 and mixed with pipette action.
3. After 30 min incubation at RT, added 100µl freshly resuspended cells (5 x 10⁴ cells/well DMEM culture medium supplemented with 10 % foetal calf serum) and mixed with pipette.
4. Duplicate 65µl aliquots of the cells + polyalkylamine/DNA complexes were immediately transferred to separate wells of a 96-well culture plate and placed in 37 °C incubator with 5 % CO₂.
5. 20 to 24 hours after initial transfection of cells, Hoechst 33258 dye was added to cells at a final concentration of 0.2 µg/ml and cells incubated for a further 30 min at 37 °C. Cells were then placed on an inverted microscope and 2 independent sets of images of the cells captured from each well as shown in **Fig. 1****.**

For each sample, 3 images were captured: bright field image of cells (**Fig. 1** upper panels), Hoechst dye stained image of total cell nuclei (**Fig. 1** middle panels) and GFP images showing cells successfully transfected with plasmid DNA and expressing GFP protein (**Fig. 1** lower panels). Microscope images of transfected HEK 293 cells, showing both the transfection efficiency and toxicity level of one of our polyalkylamine molecules (pN20HL5) compared to a commonly used commercial transfection reagent (reference reagent). Polyalkylamine reagent pN19HL5 has a transfection efficiency of approximately 95 % and has low cell toxicity (very few brightly stained apoptotic nuclei).

According to the protocol given in Example 15, a library of newly synthesized compounds according to claim 1 have been tested for their ability to transfect HEK 293 cells. The graph in **Fig. 2** shows the transfection efficiency of these polyalkylamine compounds compared to a commercially available transfection agent reference. 1 of the polyalkylamine molecules is efficient at delivering plasmid DNA (GFP gene) to HEK 293 cells when compared to a widely used commercial transfection reagent, indicated by the solid line. One reagent, in particular has been identified as possessing very low toxicity and has the ability to very efficiently deliver siRNA molecules to cells (pN19HL5; see **Fig. 3**).

### Example 16: Screen of library "hits" for ability to transfect siRNA

One of the key technologies for manipulation of gene function, both in cells and whole organisms, is gene silencing through RNA interference (RNAi). Delivery of small interfering RNA (siRNA) molecules into mammalian cells is crucial for this technology and has significant clinical/therapeutic implications.
Thus, in addition to screening our polyalkylamines for delivery of plasmid DNA (the genes) we have also screened polyalkylamines according to the present invention for their ability to efficiently deliver siRNA molecules (the gene silencers).

In order to screen for this, U87Luc cells stably express the *Photinus pyralis* luciferase were used to test the ability of our polyalkylamine reagents to deliver siRNA targeting luciferase gene.

Assay: Transfection of siRNA in U87Luc cells.

### Method:

All steps performed in 0.2ml PCR tubes and 96 well plate format. Amounts shown are for one well of a 96-well plate.
1. 0.25 µl polyalkylamine reagents diluted in 8 µl 50 mmol/l NaOAc buffer, pH 5.0.
2. Diluted polyalkylamine reagents from (1) added to 1 pmol (0.2 µl of 5 µmol/l) siRNA molecules in 8 µl NaOAc buffer, pH 5.0 and mixed with pipette action. The siRNA molecules used had either a scrambled sequence not specific for any known gene (Con siRNA), or a sequence specifically targeting the endogenous mRNA from the luciferase gene (Luc siRNA).
3. After 30 min incubation at RT, added 50 µl freshly resuspended cells (2 x 10⁴ cells/well DMEM culture medium supplemented with 10 % foetal calf serum) in one well of a 96-well plate and placed in 37 °C incubator with 5 % CO₂.
4. 48 hours after initial transfection, cells, were lysed in 40 µl (1X PLB).
5. Standard luciferase assay was performed.

### Results

**Fig. 3** shows the expression of luciferase protein from total lysates of U87Luc cells transfected with the indicated siRNA molecules and cultured for 48 hours. Polyalkylamine reagent pN19HL5 is quite effective compared with the commercial reagent A (LF2K) at siRNA mediated gene silencing. This demonstrates the striking difference in properties of related transfection reagents and highlights the importance of using our novel method to easily synthesise hundreds of related polyalkylamine that can be screened to identify the ones having optimal properties (such as highly efficient DNA and siRNA delivery as well as low cellular toxicity).

The results of the *in vitro* HEK 293T cells transfection screening are shown in figure 4. eGFP plasmids were mixed with 40 polyamines and 40 polyalkylamines to form polyplexes at one weight (µg) to molar (µmol) ratios (DNA : Polymer monomer = 75:2) and were screened in HEK293T cells. Transfection was performed with a one-step protocol, where freshly resuspended HEK293T cells were added directly to the polyplexes, followed by a transfer to 96 well plates. 20 hours later, cells were nucleus stained with Hoechst 33342, imaged for GFP expression automatically with a fluorescent microscope and analyzed for transfection efficiency by CellProfiler. Lipofectamine 2000 was used as positive control. Surprisingly, the results showed that polyalkylamines have higher transfection efficiency compared to the corresponding polyamines.

The inventors tested siRNA delivery using four potent polyalkylamine transfection reagents (pN12HL5, pN13HL5, pN19HL5 and pN20HL5) and their corresponding polyamines (pN12L5, pN13L5, pN19L5 and pN20L5) (Figure 5). LF2K was used as the positive control, and untreated cells (NC) were used as the negative control. Luc siRNA targeting mRNA for the luciferase protein and U87 Luc-GFP cell line (human glioblastoma-astrocytoma) that has been stably transformed to express a fusion protein of GFP and luciferase were used for the transfection. 8 polymer particles formed polyplexes with Luc siRNA or Con siRNA (siRNA : Polymer monomer = 2 pmol : 1 µmol) and were incubated with U87 Luc-GFP cells for 48h. Standard luciferase assay was performed to quantify the expression levels of luciferase protein in cell lysates. The result showed that polyalkylamines enhanced siRNA transfection efficiency. pN19HL5 showed excellent knockdown of luciferase protein, achieving 95 % knockdown, compared to LF2K.

Further examples of compounds according to the invention are given in the tables below.

### For polyamines:

| Amine | | Linker | | Polymer number |
|---|---|---|---|---|
| N1 | 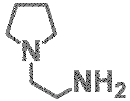 | L1 | 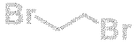 | pN1L1 |
| N2 | 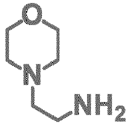 | L1 | 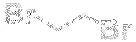 | pN2L1 |
| N3 | 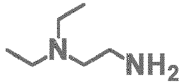 | L1 | 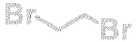 | pN3L1 |
| N4 | 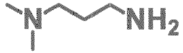 | L1 | 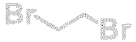 | pN4L1 |
| N5 | 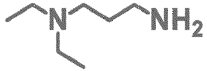 | L1 | 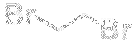 | pN5L1 |
| N6 | 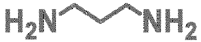 | L1 | 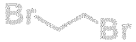 | pN6L1 |
| N7 | 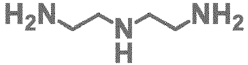 | L1 | 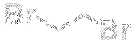 | pN7L1 |
| N8 | 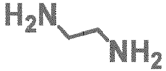 | L1 | 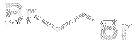 | pN8L1 |
| N9 | 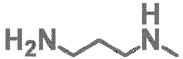 | L1 | 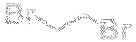 | pN9L1 |
| N10 | 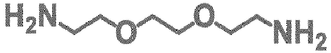 | L1 | 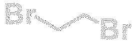 | pN10L1 |
| N11 | 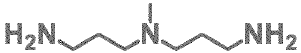 | L1 | 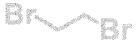 | pN11L1 |
| N12 | 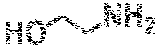 | L1 | 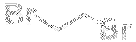 | pN12L1 |
| N13 | 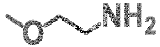 | L1 | 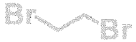 | pN13L1 |
| N14 | 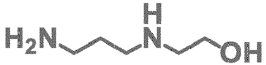 | L1 | 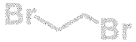 | pN14L1 |
| N15 | 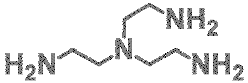 | L1 | 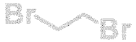 | pN15L1 |
| N16 | 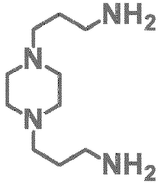 | L1 | 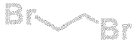 | pN16L1 |
| N17 | 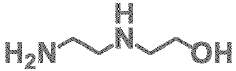 | L1 | 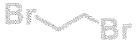 | pN17L1 |
| N18 | 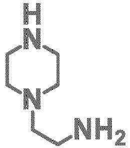 | L1 | 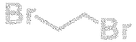 | pN18L1 |
| N19 | 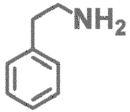 | L1 | 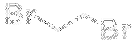 | pN19L1 |
| N20 | 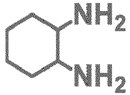 | L1 | 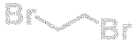 | pN20L1 |
| N1 | 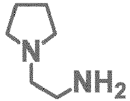 | L2 | 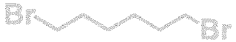 | pN1L2 |
| N2 | 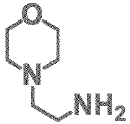 | L2 | 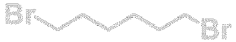 | pN2L2 |
| N3 | 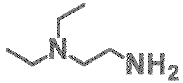 | L2 | 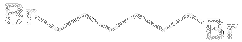 | pN3L2 |
| N4 | 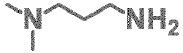 | L2 | 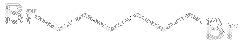 | pN4L2 |
| N5 | 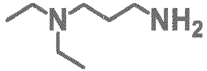 | L2 | 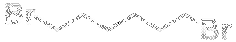 | pN5L2 |
| N6 | 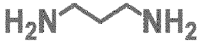 | L2 | 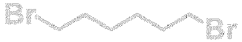 | pN6L2 |
| N7 | 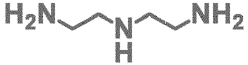 | L2 | 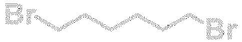 | pN7L2 |
| N8 | 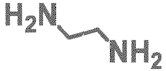 | L2 | 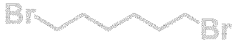 | pN8L2 |
| N9 | 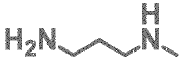 | L2 | 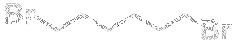 | pN9L2 |
| N10 | 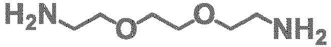 | L2 | 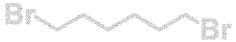 | pN10L2 |
| N11 | 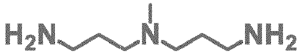 | L2 | 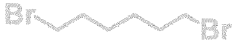 | pN11L2 |
| N12 | 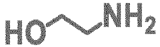 | L2 | 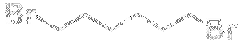 | pN12L2 |
| N13 | 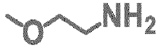 | L2 | 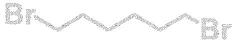 | pN13L2 |
| N14 | 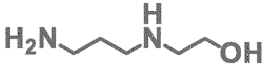 | L2 | 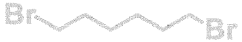 | pN14L2 |
| N15 | 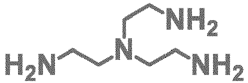 | L2 | 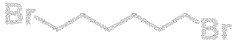 | pN15L2 |
| N16 | 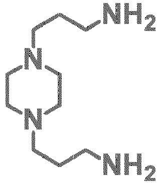 | L2 | 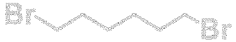 | pN16L2 |
| N17 | 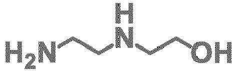 | L2 | 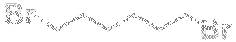 | pN17L2 |
| N18 | 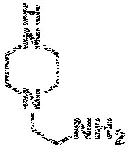 | L2 | 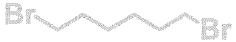 | pN18L2 |
| N19 | 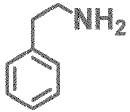 | L2 | 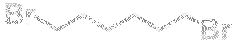 | pN19L2 |
| N20 | 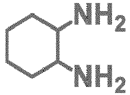 | L2 | 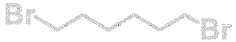 | pN20L2 |
| N1 | 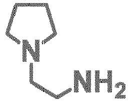 | L3 | 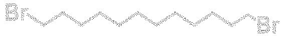 | pN1L3 |
| N2 | 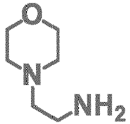 | L3 | 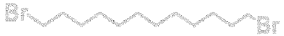 | pN2L3 |
| N3 | 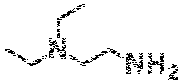 | L3 | 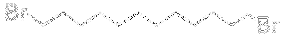 | pN3L3 |
| N4 | 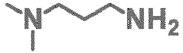 | L3 | 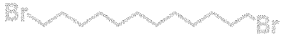 | pN4L3 |
| N5 | 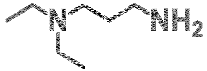 | L3 | 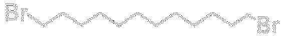 | pN5L3 |
| N6 | 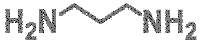 | L3 | 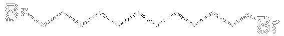 | pN6L3 |
| N7 | 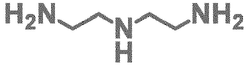 | L3 | 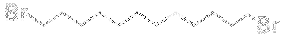 | pN7L3 |
| N8 | 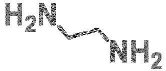 | L3 | 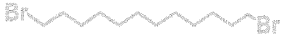 | pN8L3 |
| N9 | 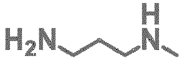 | L3 | 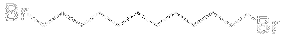 | pN9L3 |
| N10 | 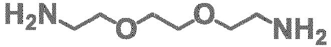 | L3 | 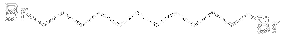 | pN10L3 |
| N11 | 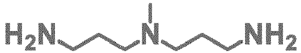 | L3 | 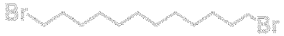 | pN11L3 |
| N12 | 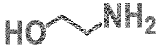 | L3 | 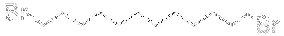 | pN12L3 |
| N13 | 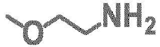 | L3 | 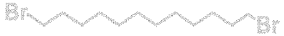 | pN13L3 |
| N14 | 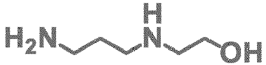 | L3 | 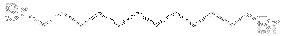 | pN14L3 |
| N15 | 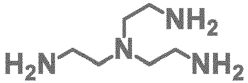 | L3 | 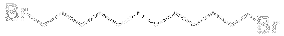 | pN15L3 |
| N16 | 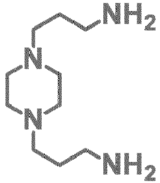 | L3 | 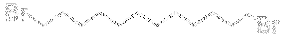 | pN16L3 |
| N17 | 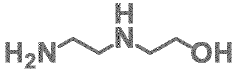 | L3 | 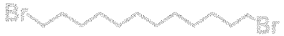 | pN17L3 |
| N18 | 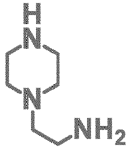 | L3 | 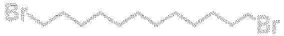 | pN18L3 |
| N19 | 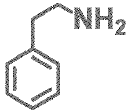 | L3 | 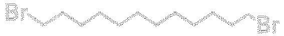 | pN19L3 |
| N20 | 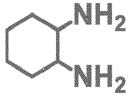 | L3 | 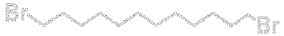 | pN20L3 |
| N4 | 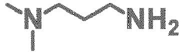 | L4 | 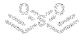 | pN4L4 |
| N5 | 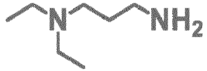 | L4 | 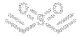 | pN5L4 |
| N15 | 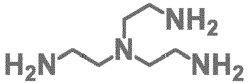 | L4 | 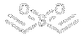 | pN15L4 |
| N1 | 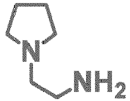 | L5 | 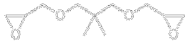 | pN1L5 |
| N2 | 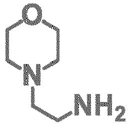 | L5 | 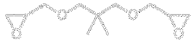 | pN2L5 |
| N3 | 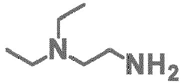 | L5 | 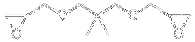 | pN3L5 |
| N4 | 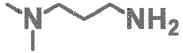 | L5 | 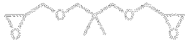 | pN4L5 |
| N5 | 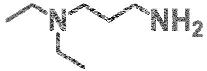 | L5 | 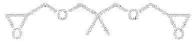 | pN5L5 |
| N6 | 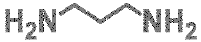 | L5 | 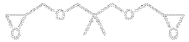 | pN6L5 |
| N7 | 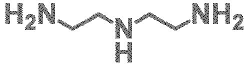 | L5 | 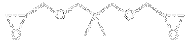 | pN7L5 |
| N8 | 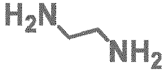 | L5 | 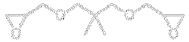 | pN8L5 |
| N9 | 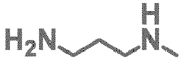 | L5 | 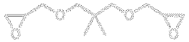 | pN9L5 |
| N10 | 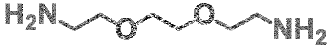 | L5 | 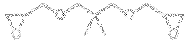 | pN10L5 |
| N11 | 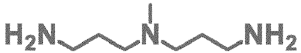 | L5 | 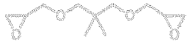 | pN11L5 |
| N12 | 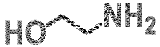 | L5 | 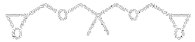 | pN12L5 |
| N13 | 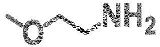 | L5 | 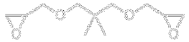 | pN13L5 |
| N14 | 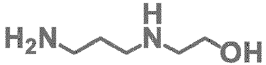 | L5 | 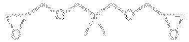 | pN14L5 |
| N15 | 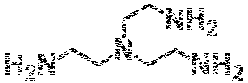 | L5 | 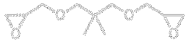 | pN15L5 |
| N16 | 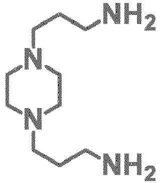 | L5 | 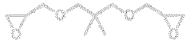 | pN16L5 |
| N17 | 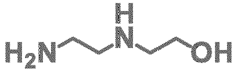 | L5 | 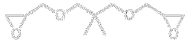 | pN17L5 |
| N18 | 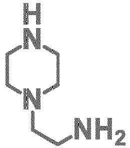 | L5 | 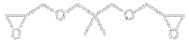 | pN18L5 |
| N19 | 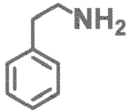 | L5 | 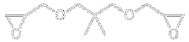 | pN19L5 |
| N20 | 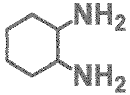 | L5 | 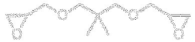 | pN20L5 |
| N1 | 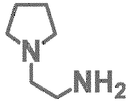 | L6 | 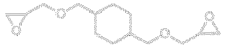 | pN1L6 |
| N2 | 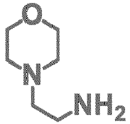 | L6 | 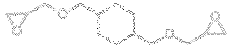 | pN2L6 |
| N3 | 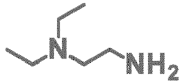 | L6 | 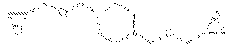 | pN3L6 |
| N4 | 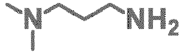 | L6 | 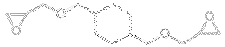 | pN4L6 |
| N5 | 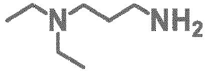 | L6 | 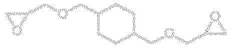 | pN5L6 |
| N6 | 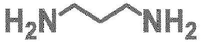 | L6 | 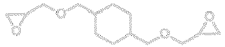 | pN6L6 |
| N7 | 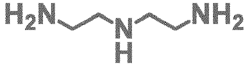 | L6 | 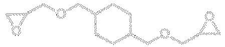 | pN7L6 |
| N8 | 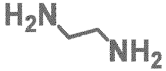 | L6 | 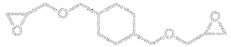 | pN8L6 |
| N9 | 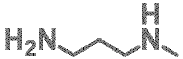 | L6 | 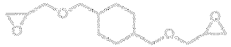 | pN9L6 |
| N10 | 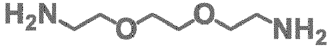 | L6 | 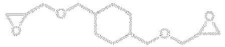 | pN10L6 |
| N11 | 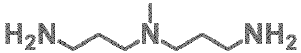 | L6 | 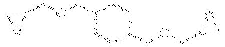 | pN11L6 |
| N12 | 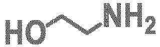 | L6 | 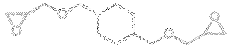 | pN12L6 |
| N13 | 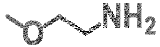 | L6 | 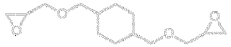 | pN13L6 |
| N14 | 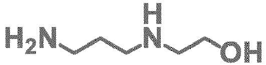 | L6 | 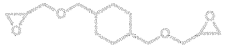 | pN14L6 |
| N15 | 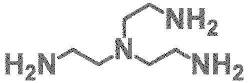 | L6 | 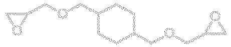 | pN15L6 |
| N16 | 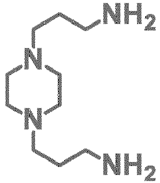 | L6 | 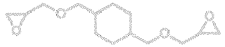 | pN16L6 |
| N17 | 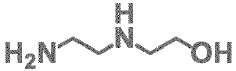 | L6 | 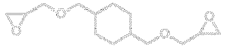 | pN17L6 |
| N18 | 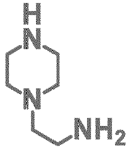 | L6 | 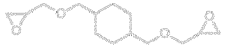 | pN18L6 |
| N19 | 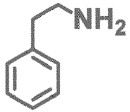 | L6 | 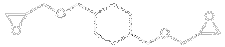 | pN19L6 |
| N20 | 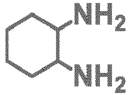 | L6 | 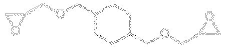 | pN20L6 |

### For polyalkylamines:

Alkyl amine (H): **H₂N** 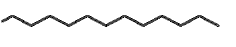

| Amine | | Alkyl amine | Linker | | Polymer number |
|---|---|---|---|---|---|
| N1 | 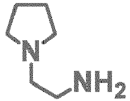 | H | L1 | 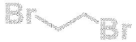 | pN1HL1 |
| N2 | 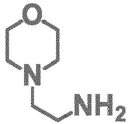 | H | L1 | 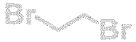 | pN2HL1 |
| N3 | 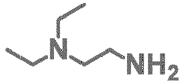 | H | L1 | 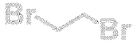 | pN3HL1 |
| N4 | 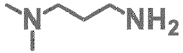 | H | L1 | 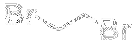 | pN4HL1 |
| N5 | 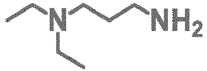 | H | L1 | 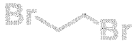 | pN5HL1 |
| N6 | 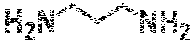 | H | L1 | 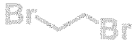 | pN6HL1 |
| N7 | 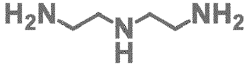 | H | L1 | 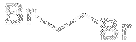 | pN7HL1 |
| N8 | 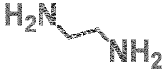 | H | L1 | 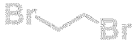 | pN8HL1 |
| N9 | 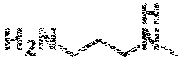 | H | L1 | 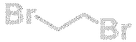 | pN9HL1 |
| N10 | 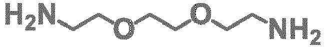 | H | L1 | 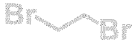 | pN10HL1 |
| N11 | 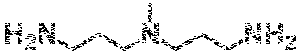 | H | L1 | 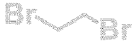 | pN11HL1 |
| N12 | 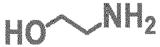 | H | L1 | 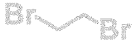 | pN12HL1 |
| N13 | 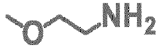 | H | L1 | 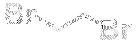 | pN13HL1 |
| N14 | 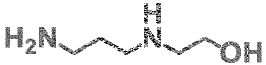 | H | L1 | 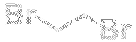 | pN14HL1 |
| N15 | 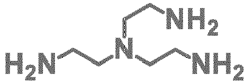 | H | L1 | 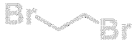 | pN15HL1 |
| N16 | 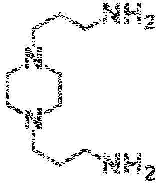 | H | L1 | 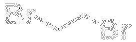 | pN16HL1 |
| N17 | 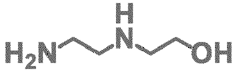 | H | L1 | 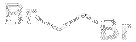 | pN17HL1 |
| N18 | 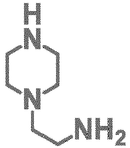 | H | L1 | 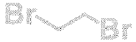 | pN18HL1 |
| N19 | 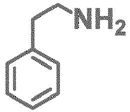 | H | L1 | 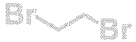 | pN19HL1 |
| N20 | 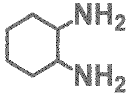 | H | L1 | 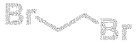 | pN20HL1 |
| N1 | 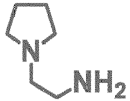 | H | L2 | 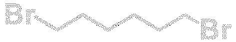 | pN1HL2 |
| N2 | 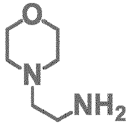 | H | L2 | 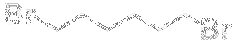 | pN2HL2 |
| N3 | 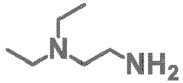 | H | L2 | 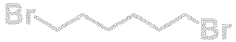 | pN3HL2 |
| N4 | 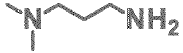 | H | L2 | 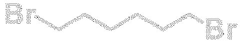 | pN4HL2 |
| N5 | 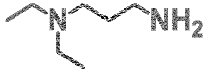 | H | L2 | 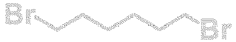 | pN5HL2 |
| N6 | 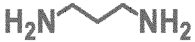 | H | L2 | 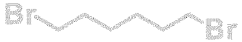 | pN6HL2 |
| N7 | 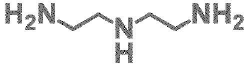 | H | L2 | 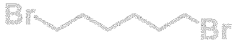 | pN7HL2 |
| N8 | 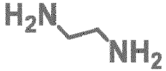 | H | L2 | 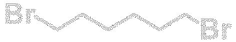 | pN8HL2 |
| N9 | 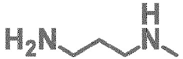 | H | L2 | 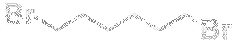 | pN9HL2 |
| N10 | 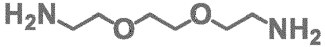 | H | L2 | 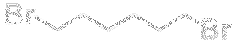 | pN10HL2 |
| N11 | 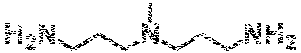 | H | L2 | 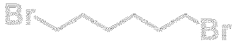 | pN11HL2 |
| N12 | 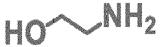 | H | L2 | 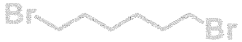 | pN12HL2 |
| N13 | 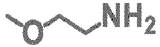 | H | L2 | 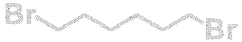 | pN13HL2 |
| N14 | 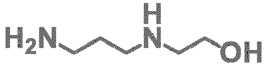 | H | L2 | 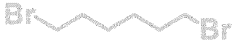 | pN14HL2 |
| N15 | 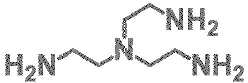 | H | L2 | 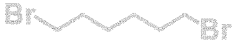 | pN15HL2 |
| N16 | 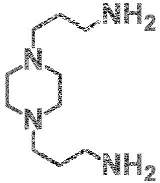 | H | L2 | 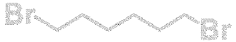 | pN16HL2 |
| N17 | 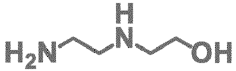 | H | L2 | 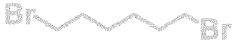 | pN17HL2 |
| N18 | 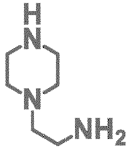 | H | L2 | 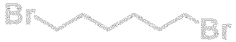 | pN18HL2 |
| N19 | 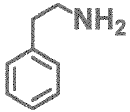 | H | L2 | 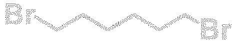 | pN19HL2 |
| N20 | 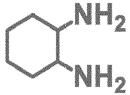 | H | L2 | 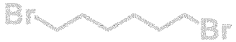 | pN20HL2 |
| N1 | 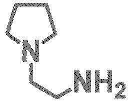 | H | L3 | 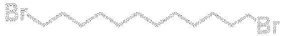 | pN1HL3 |
| N2 | 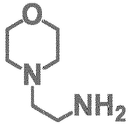 | H | L3 | 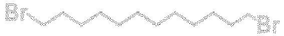 | pN2HL3 |
| N3 | 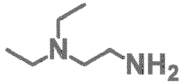 | H | L3 | 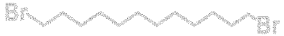 | pN3HL3 |
| N4 | 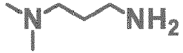 | H | L3 | 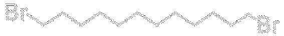 | pN4HL3 |
| N5 | 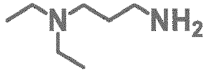 | H | L3 | 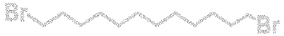 | pN5HL3 |
| N6 | 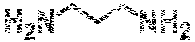 | H | L3 | 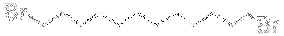 | pN6HL3 |
| N7 | 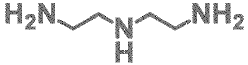 | H | L3 | 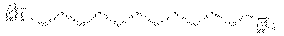 | pN7HL3 |
| N8 | 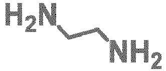 | H | L3 | 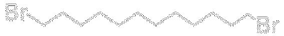 | pN8HL3 |
| N9 | 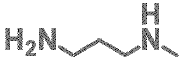 | H | L3 | 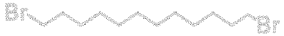 | pN9HL3 |
| N10 | 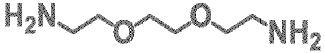 | H | L3 | 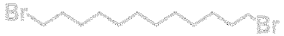 | pN10HL3 |
| N11 | 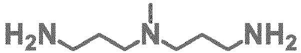 | H | L3 | 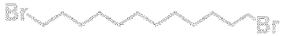 | pN11HL3 |
| N12 | 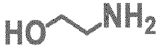 | H | L3 | 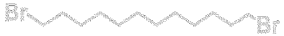 | pN12HL3 |
| N13 | 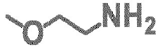 | H | L3 | 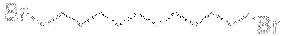 | pN13HL3 |
| N14 | 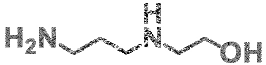 | H | L3 | 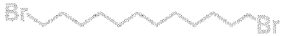 | pN14HL3 |
| N15 | 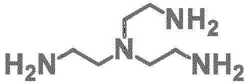 | H | L3 | 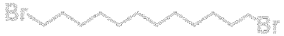 | pN15HL3 |
| N16 | 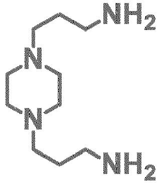 | H | L3 | 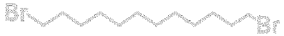 | pN16HL3 |
| N17 | 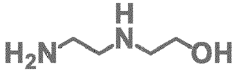 | H | L3 | 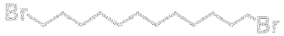 | pN17HL3 |
| N18 | 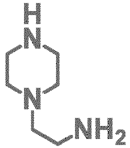 | H | L3 | 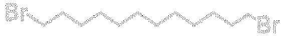 | pN18HL3 |
| N19 | 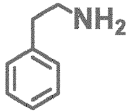 | H | L3 | 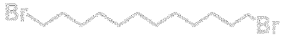 | pN19HL3 |
| N20 | 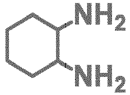 | H | L3 | 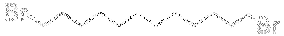 | pN20HL3 |
| N4 | 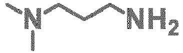 | H | L4 | 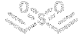 | pN4HL4 |
| N5 | 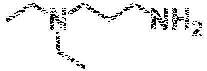 | H | L4 | 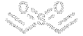 | pN5HL4 |
| N15 | 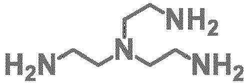 | H | L4 | 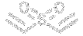 | pN15HL4 |
| N1 | 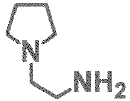 | H | L5 | 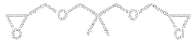 | pN1HL5 |
| N2 | 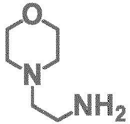 | H | L5 | 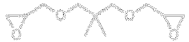 | pN2HL5 |
| N3 | 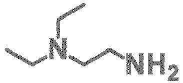 | H | L5 | 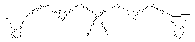 | pN3HL5 |
| N4 | 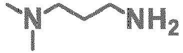 | H | L5 | 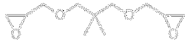 | pN4HL5 |
| N5 | 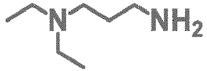 | H | L5 | 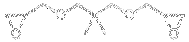 | pN5HL5 |
| N6 | 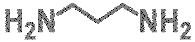 | H | L5 | 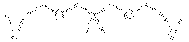 | pN6HL5 |
| N7 | 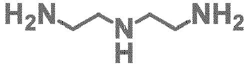 | H | L5 | 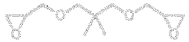 | pN7HL5 |
| N8 | 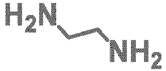 | H | L5 | 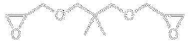 | pN8HL5 |
| N9 | 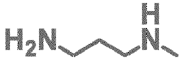 | H | L5 | 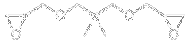 | pN9HL5 |
| N10 | 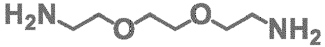 | H | L5 | 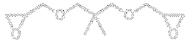 | pN10HL5 |
| N11 | 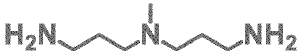 | H | L5 | 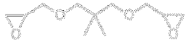 | pN11HL5 |
| N12 | 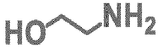 | H | L5 | 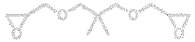 | pN12HL5 |
| N13 | 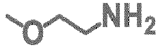 | H | L5 | 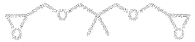 | pN13HL5 |
| N14 | 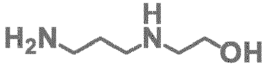 | H | L5 | 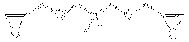 | pN14HL5 |
| N15 | 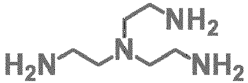 | H | L5 | 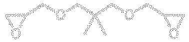 | pN15HL5 |
| N16 | 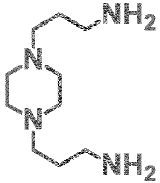 | H | L5 | 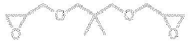 | pN16HL5 |
| N17 | 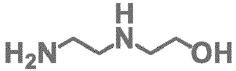 | H | L5 | 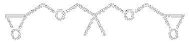 | pN17HL5 |
| N18 | 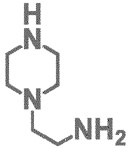 | H | L5 | 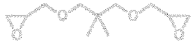 | pN18HL5 |
| N19 | 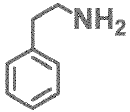 | H | L5 | 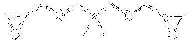 | pN19HL5 |
| N20 | 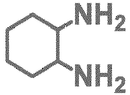 | H | L5 | 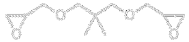 | pN20HL5 |
| N1 | 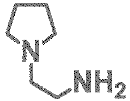 | H | L6 | 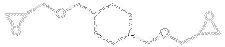 | pN1HL6 |
| N2 | 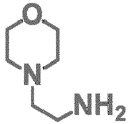 | H | L6 | 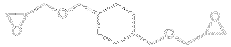 | pN2HL6 |
| N3 | 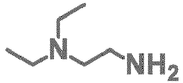 | H | L6 | 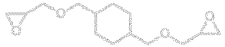 | pN3HL6 |
| N4 | 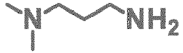 | H | L6 | 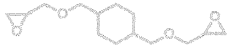 | pN4HL6 |
| N5 | 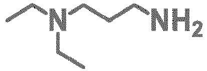 | H | L6 | 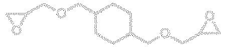 | pN5HL6 |
| N6 | 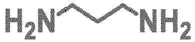 | H | L6 | 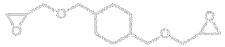 | pN6HL6 |
| N7 | 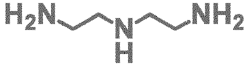 | H | L6 | 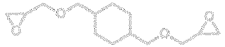 | pN7HL6 |
| N8 | 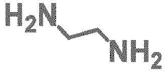 | H | L6 | 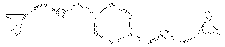 | pN8HL6 |
| N9 | 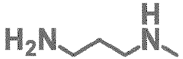 | H | L6 | 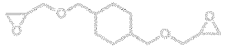 | pN9HL6 |
| N10 | 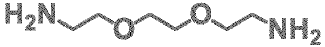 | H | L6 | 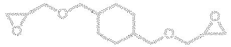 | pN10HL6 |
| N11 | 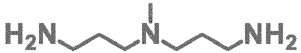 | H | L6 | 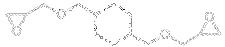 | pN11HL6 |
| N12 | 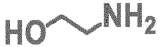 | H | L6 | 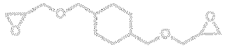 | pN12HL6 |
| N13 | 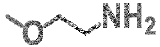 | H | L6 | 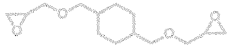 | pN13HL6 |
| N14 | 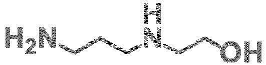 | H | L6 | 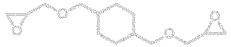 | pN14HL6 |
| N15 | 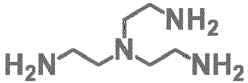 | H | L6 | 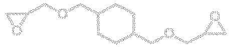 | pN15HL6 |
| N16 | 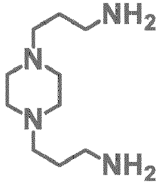 | H | L6 | 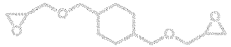 | pN16HL6 |
| N17 | 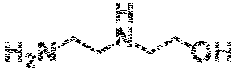 | H | L6 | 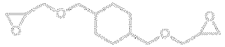 | pN17HL6 |
| N18 | 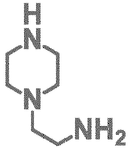 | H | L6 | 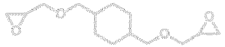 | pN18HL6 |
| N19 | 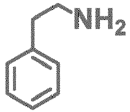 | H | L6 | 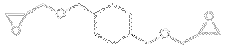 | pN19HL6 |
| N20 | 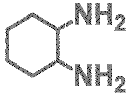 | H | L6 | 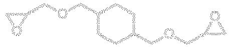 | pN20HL6 |

### List of abbreviations

- Ar: Argon
- DCM: Dichloromethane
- DIC: N, N'-Dlllsopropylcarbodlllmide
- DMEM: Culture medium
- DMF: Dimethylformamide
- DNA: Desoxyribonucleic acid
- DOPE: Dioleolylphosphatidylethanolamine
- GFP: Green Fluorescent Protein
- kD: kilo Dalton
- U87Luc: U87 epithelial-like cell line (human glioblastoma-astrocytoma)
- PEG: Polyethylene glycol
- RNA: Ribonucleic acid
- RNAi: RNA interference
- siRNA: small interfering RNA
- THF: Tetrahydrofuran

## Claims

1. Polyalkylamine comprising a structure of the following formula (I), 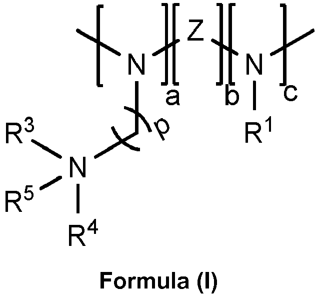 wherein
a and b are integers from 1 to 100 and c is an integer from 0 to 100;.
R¹ is C₁-C₂₄ alkyl, C₂-C₂₄ alkenyl, C₂-C₂₄ alkynyl, or C₆-C₂₄ acyl, which can be optionally substituted with a C₁-C₆ hydrocarbyl group;
Z is either C1-C12 alkyls or S(=O)₂ or 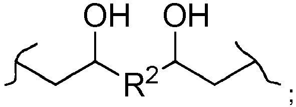 squiggle line stand for a connection to the rest of the structure;
R² is either a mono, poly or cyclic ether or an ether which is optionally substituted with at least one C1-C6 hydrocarbyl group, or with at least one hydroxy group;
R³ and R⁴ are either the same or different and independently C₁-C₁₂ alkyl, C₁-C₁₂ alkenyl, or C₁-C₁₂ alkynyl, wherein alkyl, alkenyl or alkynyl is optionally substituted with at least one C₁-C₆ hydrocarbyl group, or R³ and R⁴ may join to form, together with the nitrogen atom to which they are bound, an optionally substituted heterocyclic ring of 3 to 10 atoms and 0 to 6 additional heteroatoms chosen from nitrogen, thiol and oxygen;
R³ and R⁴ can also connect two or more polymer chains of a structure of Formula (I) leading to cross-linked or branched polymers;
R⁵ is either absent or is hydrogen or C₁-C₁₂ alkyl to provide a quaternary amine;
p is an integer from 1 to 12.

2. Polyalkylamine of claim 1, wherein R¹ is C₆-C₂₄ alkyl, which is optionally substituted with a C₁-C₆ hydrocarbyl group.

3. Polyalkylamine of claim 1 or 2, comprising a structure of formula (III) 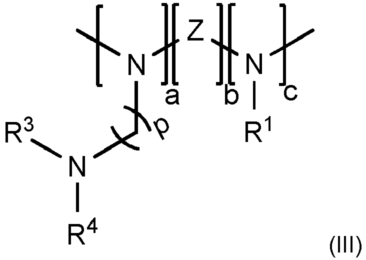 wherein
a and b are integers from 1 to 100 and c is an integer from 0 to 100;
R¹ is C₆-C₁₈ alkyl;
R³ and R⁴ are either the same or different C₁-C₁₂ alkyls;
wherein alkyl may be optionally substituted with a C₁-C₆ hydrocarbyl group;
or R³ and R⁴ may join to form, together with the nitrogen atom to which they are bound, an optionally substituted heterocyclic ring of 3 to 10 atoms comprising 1 to 7 heteroatoms chosen from nitrogen, thiol and oxygen; wherein R³ and R⁴ can also connect two or more polymer chains comprising a structure of Formula (III) leading to cross-linked or branched polymers;
p is an integer from 1 to 12.

4. Polyalkylamine of claims 1 to 3, comprising the structure of formula (III), 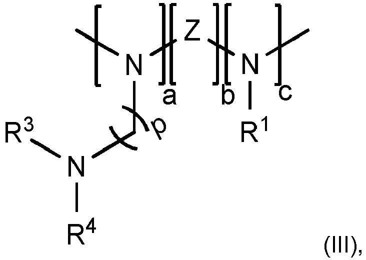 wherein
a and b are integers from 1 to 100 and c is an integer from 0 to 100;
R¹ is C₁₁-C₁₂ alkyl,
R³ and R⁴ are the same C₁-C₂ alkyl, R³ and R⁴ can also connect two or more polymer chains comprising a structure of Formula (III) leading to cross-linked or branched polymers, p is an integer from 1 to 2.

5. Polyalkylamine of claims 1 to 4, wherein c is 0.

6. A method for synthesizing a polyalkylamine of claims 1 to 5, comprising the step of:
reacting of dihalides, dialkenes or diepoxides of the general formula (IVa), (IVb), or (IVc) 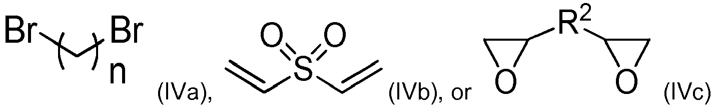 with an amine of the general formula (R³R⁴R⁵N)(CH₂)ₚX, and with an amine of the general formula, NH₂-R¹,
under polymerization to yield a compound of the general formula (Va), (Vb) or (Vc) 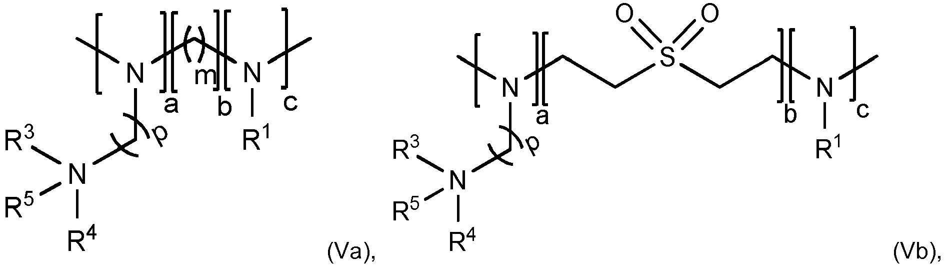 or 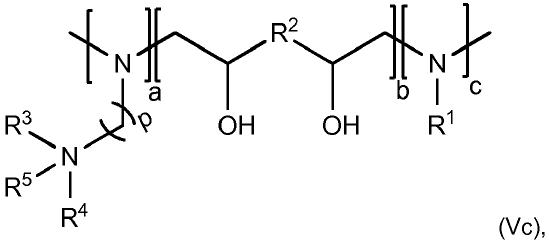 wherein
X is NH₂ or NH.
m is an integer from 1 to 12;
and
wherein a, b, c, n, p, R¹, R², R³, R⁴ and R⁵ are defined as in claim 1.

7. A polymer particle containing a polyalkylamine of claims 1 to 5.

8. Polymer particle according to claim 7, wherein said polymer particle is a polymersome.

9. Polymer particle according to claim 7 or 8, further containing a non-cationic lipid, a sterol, and/or a biorective agent.

10. Polymer particle according to claim 9, wherein said bioactive agent is selected from the group consisting of: a nucleic acid, an antineoplastic agent, an antibiotic, an immunomodulator, an anti-inflammatory agent, an agent acting on the central nervous system, a polypeptide and a polypeptoid.

11. In vitro use of a polymer particle according to one of the claims 7 to 10 for delivering a bioactive agent into a cell.

12. Polymer particle according to claims 7 to 10 for use as a medicament, in particular for treating a viral infection, a liver disease or disorder, or cancer.

13. A kit, comprising at least 5, in particular at least 10, at least 100, at least 1000, or at least at least 10.000 different polyalkylamines of claims 1 to 5.

14. Use of a kit of claim 13 for screening a plurality of different polyalkylamine of claims 1 to 5 for a desired property.

15. A method for screening a plurality of different polyalkylamine of claims 1 to 5 for a desired property, comprising bringing the plurality of different polyalkylamines into contact with a given type of cell and determining which of the polyalkylamine has the desired property.

## Patentansprüche

1. Polyalkylamin, aufweisend eine Struktur gemäß der folgenden Formel (I), 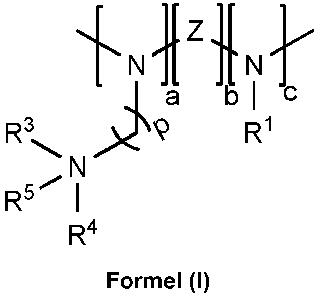 wobei
a und b ganze Zahlen von 1 bis 100 sind und c eine ganze Zahl von 0 bis 100 ist;
R¹ ein C₁-C₂₄ Alkyl, C₂-C₂₄ Alkenyl, C₂-C₂₄ Alkinyl, oder C₆-C₂₄ Acyl ist, das optional mit einer C₁-C₆ Kohlenwasserstoffgruppe substituiert ist;
Z ist entweder ein C1-C12 Alkyl oder S(=O)₂ oder 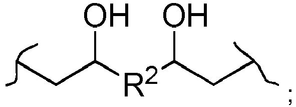 wobei die gewellte Linie für eine Verbindung zum Rest der Struktur steht;
R² ist entweder ein monomerer, polymerer oder cyclischer Ether oder ein Ether, der optional mit mindestens einer C1-C6 Kohlenwasserstoffgruppe substituiert ist, oder mit mindestens einer Hydroxygruppe;
R³ und R⁴ entweder identisch oder unterschiedlich und unabhängig voneinander C₁-C₁₂ Alkyl, C₁-C₁₂ Alkenyl oder C₁-C₁₂ Alkinyl sind, wobei Alkyl, Alkenyl oder Alkinyl optional substituiert sind mit mindestens einer C₁-C₆ Kohlenwasserstoffgruppe, oder R³ und R⁴ miteinander verbunden sind, um zusammen mit dem Stickstoffatom, an dem sie gebunden sind, einen optional substituierten heterocyclischen Ring aus 3 bis 10 Atomen und 0 bis 6 zusätzlichen Heteroatomen ausgewählt aus Stickstoff, Thiol und Sauerstoff, zu bilden;
R³ and R⁴ können auch zwei oder mehrere Polymerketten mit einer Struktur der Formel (I) verbinden, was zu quervernetzten oder verzweigten Polymeren führt;
R⁵ ist entweder nicht vorhanden oder ist Wasserstoff oder ein C₁-C₁₂ Alkyl, um ein quartäres Amin bereitzustellen;
p ist eine ganze Zahl von 1 bis 12.

2. Polyalkylamin gemäß Anspruch 1, wobei R¹ ein C₆-C₂₄ Alkyl ist, das optional mit einer C₁-C₆ Kohlenwasserstoffgruppe substituiert ist.

3. Polyalkylamin nach Anspruch 1 oder 2, aufweisend eine Struktur gemäß Formel (III) 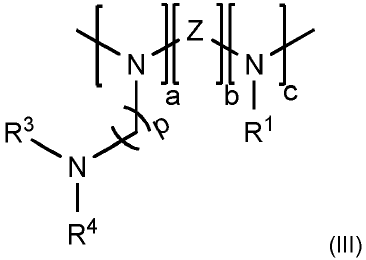 wobei
a und b ganze Zahlen von 1 bis 100 sind und c eine ganze Zahl von 0 bis 100 ist;
R¹ ein C₆-C₁₈ Alkyl ist;
R³ und R⁴ entweder identische oder unterschiedliche C₁-C₁₂ Alkyl sind;
wobei Alkyl optional mit einer C₁-C₆ Kohlenwasserstoffgruppe substituiert ist;
oder R³ und R⁴ miteinander verbunden sind, um zusammen mit dem Stickstoffatom, an dem sie gebunden sind, einen optional substituierten heterocyclischen Ring aus 3 bis 10 Atoms aufweisend 1 bis 7 Heteroatome ausgewählt von Stickstoff, Thiol und Sauerstoff, zu bilden;
wobei R³ und R⁴ auch zwei oder mehrere Polymerketten aufweisend eine Struktur der Formel (III) verbinden können, was zu einem quervernetzten verzweigten Polymer führt;
p ist eine ganze Zahl von 1 bis 12.

4. Polyalkylamin nach Anspruch 1 bis 3, aufweisend eine Struktur gemäß Formel (III), 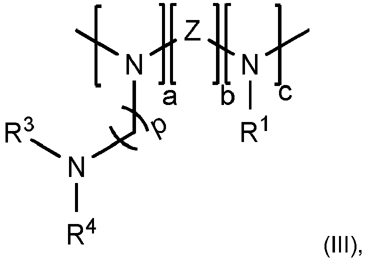 wobei
a und b ganze Zahlen von 1 bis 100 sind und c eine ganze Zahl von 0 bis 100 ist;
R¹ ein C₁₁-C₁₂ Alkyl ist;
R³ und R⁴ dasselbe C₁-C₂ Alkyl sind, R³ und R⁴ können auch zwei oder mehrere Polymerketten aufweisend eine Struktur gemäß Formel (III) verbinden, was zu einem quervernetzten verzweigten Polymer führt;
p eine ganze Zahl von 1 bis 12 ist.

5. Polyalkylamin nach Anspruch1 bis 4, wobei c gleich 0 ist.

6. Verfahren zur Synthese eines Polyalkylamins nach Anspruch 1 bis 5, aufweisend den Schritt der:
Reaktion von Dihalogenen, Dialkenen oder Diepoxiden gemäß der allgemeinen Formel (IVa), (IVb), oder (IVc) 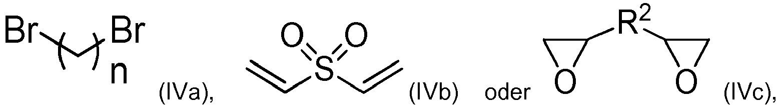 mit einem Amin der allgemeinen Formel (R³R⁴R⁵N)(CH₂)ₚX, und mit einem Amin gemäß der allgemeinen Formel NH₂-R¹,
unter Polymerisation, um eine Verbindung der allgemeinen Formel (Va), (Vb) or (Vc) zu erhalten 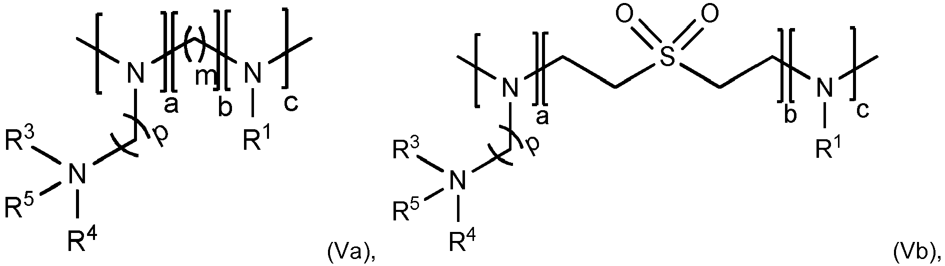 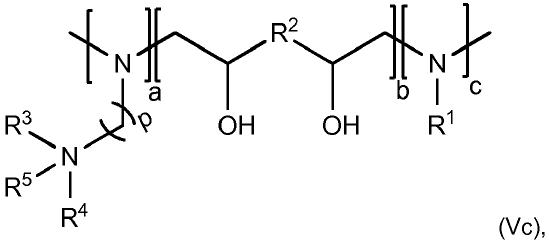 wobei
X gleich NH₂ oder NH ist.
m eine ganze Zahl von 1 bis 12 ist;
und
wobei a, b, c, n, p, R¹, R², R³, R⁴ and R⁵ wie in Anspruch 1 definiert sind.

7. Polymerpartikel aufweisend ein Polyalkylamin nach Anspruch 1 bis 5.

8. Polymerpartikel nach Anspruch 7, wobei das Polymerpartikel ein Polymersom ist.

9. Polymerpartikel nach Anspruch 7 oder 8, weiterhin aufweisend ein nicht kationisches Lipid, ein Sterol und/oder ein bioaktives Mittel.

10. Polymerartikel nach Anspruch 9, wobei das bioaktives Mittel ausgewählt ist aus der Gruppe bestehend aus: einer Nukleinsäure, einem anti-neoplasmatischem Mittel, einem Antibiotikum, einem Immunmodulator, einem entzündungshemmenden Mittel, einem am zentralen Nervensystem agierenden Mittel, einem Polypeptid und einem Polypeptoid.

11. In vitro Verwendung eines Polymerpartikels nach einem der Ansprüche 7 bis 10 zum Zuführen eines bioaktiven Mittels in eine Zelle.

12. Polymerpartikel nach Anspruch 7 bis 10 zur Verwendung als Medikament, insbesondere für die Behandlung eines viralen Infektes, einer Lebererkrankung oder einer Leberfehlfunktion oder Krebs.

13. Kit, aufweisend mindestens 5, insbesondere mindestens 10, mindestens 100, mindestens 1000 oder mindestens 10.000 verschiedene Polyalkylamine nach Anspruch 1 bis 5.

14. Verwendung eines Kits nach Anspruch 13 für das Durchtesten einer Vielzahl von verschiedenen Polyalkylaminen der Ansprüche 1 bis 5 auf eine erwünschte Eigenschaft.

15. Verfahren für das Durchtesten einer Vielzahl von verschiedenen Polyalkylaminen nach Anspruch 1 bis 5 auf eine erwünschte Eigenschaft, aufweisend das in Kontakt bringen der Vielzahl von verschiedenen Polyalkylaminen mit einer gegebenen Zellart und der Bestimmung, welche der Polyalkylamine die gewünschte Eigenschaft hat.

## Revendications

1. Polyalkylamine comprenant une structure de la formule (I) suivante, 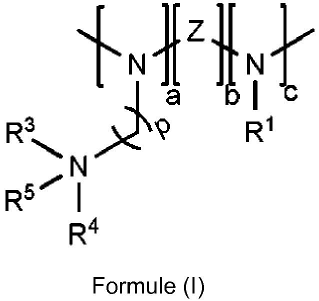 dans laquelle
a et b sont des entiers de 1 à 100 et c est un entier de 0 à 100 ;
R¹ est alkyle en C₁-C₂₄, alcényle en C₂-C₂₄, alcynyle en C₂-C₂₄, ou acyle en C₆-C₂₄, qui peut être facultativement substitué par un groupe hydrocarbyle en C₁-C₆ ;
Z est alkyle en C₁-C₁₂ ou S(=O)₂ ou 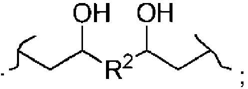 le trait irrégulier représente une liaison au reste de la structure ;
R² est un monoéther, un polyéther ou un éther cyclique ou un éther qui est facultativement substitué par au moins un groupe hydrocarbyle en C₁-C₆, ou par au moins un groupe hydroxy ;
R³ et R⁴ sont identiques ou différents et indépendamment alkyle en C₁-C₁₂, alcényle en C₁-C₁₂ ou alcynyle en C₁-C₁₂, où l'alkyle, alcényle ou alcynyle est facultativement substitué par au moins un groupe hydrocarbyle en C₁-C₆, ou R³ et R⁴ peuvent être assemblés pour former, conjointement avec l'atome d'azote auquel ils sont liés, un cycle hétérocyclique facultativement substitué de 3 à 10 atomes et 0 à 6 hétéroatomes additionnels choisis parmi azote, thiol et oxygène ;
R³ et R⁴ peuvent également relier deux ou plus de deux chaînes de polymère d'une structure de formule (I) pour obtenir des polymères réticulés ou ramifiés ;
R⁵ est absent ou est hydrogène ou alkyle en C₁-C₁₂ pour obtenir une amine quaternaire ;
p est un entier de 1 à 12.

2. Polyalkylamine de la revendication 1, dans laquelle R¹ est alkyle en C₆-C₂₄, qui est facultativement substitué par un groupe hydrocarbyle en C₁-C₆.

3. Polyalkylamine de la revendication 1 ou 2, comprenant une structure de formule (III) 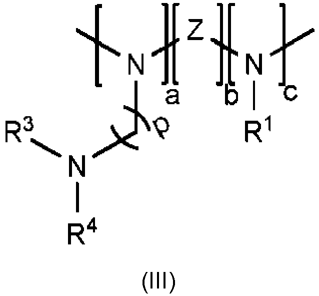 dans laquelle
a et b sont des entiers de 1 à 100 et c est un entier de 0 à 100 ;
R¹ est alkyle en C₆-C₁₈ ;
R³ et R⁴ sont des alkyles en C₁-C₁₂ identiques ou différents ;
dans laquelle un alkyle peut être facultativement substitué par un groupe hydrocarbyle en C₁-C₆ ;
ou R³ et R⁴ peuvent être assemblés pour former, conjointement avec l'atome d'azote auquel ils sont liés, un cycle hétérocyclique facultativement substitué de 3 à 10 atomes comprenant 1 à 7 hétéroatomes choisis parmi azote, thiol et oxygène ; dans laquelle R³ et R⁴ peuvent également relier deux ou plus de deux chaînes de polymère comprenant une structure de formule (III) pour obtenir des polymères réticulés ou ramifiés ;
p est un entier de 1 à 12.

4. Polyalkylamine des revendications 1 à 3, comprenant la structure de formule (III), 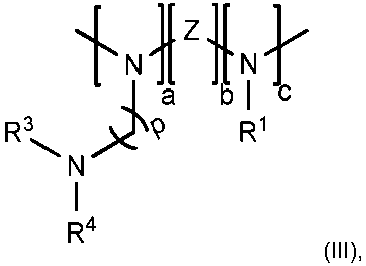 dans laquelle
a et b sont des entiers de 1 à 100 et c est un entier de 0 à 100 ;
R¹ est alkyle en C₁₁-C₁₂,
R³ et R⁴ sont le même alkyle en C₁-C₂, R³ et R⁴ peuvent également relier deux ou plus de deux chaînes de polymère comprenant une structure de formule (III) pour obtenir des polymères réticulés ou ramifiés, p est un entier de 1 à 2.

5. Polyalkylamine des revendications 1 à 4, dans lequel c est 0.

6. Procédé de synthèse d'une polyalkylamine des revendications 1 à 5, comprenant l'étape de :
réaction de dihalogénures, dialcènes ou diépoxydes de formule générale (IVa), (IVb) ou (IVc) 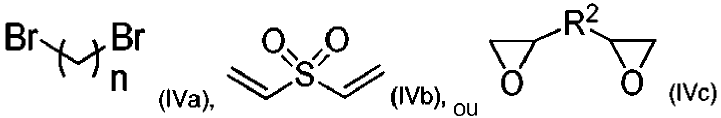 avec une amine de formule générale (R³R⁴R⁵N)(CH₂)_{P}X, et avec une amine de formule générale, NH₂-R¹,
dans des conditions de polymérisation pour obtenir un composé de formule générale (Va), (Vb) ou (Vc) 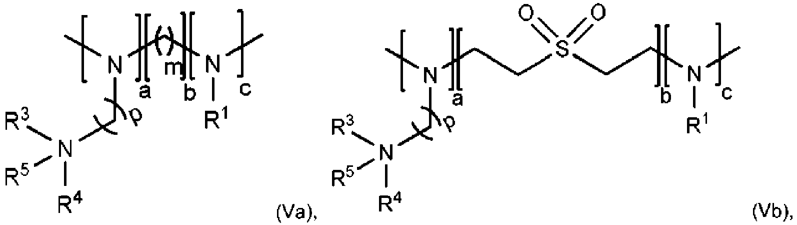 ou 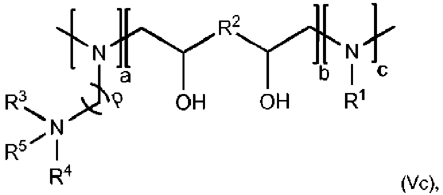 dans lequel
X est NH₂ ou NH ;
m est un entier de 1 à 12 ;
et
dans lequel a, b, c, n, p, R¹, R², R³, R⁴ et R⁵ sont tels que définis dans la revendication 1.

7. Particule de polymère contenant une polyalkylamine des revendications 1 à 5.

8. Particule de polymère selon la revendication 7, ladite particule de polymère étant un polymersome.

9. Particule de polymère selon la revendication 7 ou 8, contenant en outre un lipide non cationique, un stérol et/ou un agent bioréactif.

10. Particule de polymère selon la revendication 9, dans laquelle ledit agent bioactif est choisi dans le groupe constitué de : un acide nucléique, un agent antinéoplasique, un antibiotique, un immunomodulateur, un agent anti-inflammatoire, un agent agissant sur le système nerveux central, un polypeptide et un polypeptoïde.

11. Utilisation in vitro d'une particule de polymère selon l'une des revendications 7 à 10 pour administrer un agent bioactif dans une cellule.

12. Particule de polymère selon les revendications 7 à 10 pour utilisation en tant que médicament, en particulier pour traiter une infection virale, une maladie ou un trouble hépatique, ou un cancer.

13. Kit, comprenant au moins 5, en particulier au moins 10, au moins 100, au moins 1000, ou au moins 10 000 polyalkylamines différentes des revendications 1 à 5.

14. Utilisation d'un kit de la revendication 13 pour cribler une pluralité de polyalkylamines différentes des revendications 1 à 5 pour une propriété souhaitée.

15. Procédé de criblage d'une pluralité de polyalkylamines différentes des revendications 1 à 5 pour une propriété souhaitée, comprenant la mise en contact de la pluralité de polyalkylamines différentes avec un type de cellule donné et la détermination de la polyalkylamine qui présente la propriété souhaitée.
